(19) Europäisches Patentamt
European Patent Office
Office européen des brevets

(11) Veröffentlichungsnummer: **0 041 476**
**B1**

(12) # EUROPÄISCHE PATENTSCHRIFT

(45) Veröffentlichungstag der Patentschrift: **24.07.85**

(21) Anmeldenummer: **81810195.8**

(22) Anmeldetag: **22.05.81**

(51) Int. Cl.⁴: **C 07 C 93/08,** C 07 D 295/08, C 07 C 103/28, C 07 D 213/30, C 07 D 213/61, C 07 D 333/16, C 07 C 125/065, C 07 C 123/00, C 07 C 103/38, A 01 N 33/04, A 01 N 43/10

(54) **Neue Aryl-Phenyl-Acetylen-Verbindungen.**

(30) Priorität: **31.05.80 CH 4232/80**

(43) Veröffentlichungstag der Anmeldung: **09.12.81 Patentblatt 81/49**

(45) Bekanntmachung des Hinweises auf die Patenterteilung: **24.07.85 Patentblatt 85/30**

(84) Benannte Vertragsstaaten: **BE CH DE FR GB IT LI NL**

(56) Entgegenhaltungen:
**EP - A - 0 004 066**
**CA - A - 91 098**
**CA - A - 147 456**
**CH - A - 601 151**
**GB - A - 2 065 647**
**US - A - 4 210 610**
**US - A - 4 223 172**

**CHEMICAL ABSTRACTS, Band 93, Nr. 1, 7. Juli 1980, Seite 213, Zusammenfassung 2102d Columbus, Ohio, U.S.A. B.J. WRIGHT et al.: "Synthesis of potential herbicides designed to uncouple photophosphorylation"**

(73) Patentinhaber: **CIBA-GEIGY AG, Postfach, CH-4002 Basel (CH)**

(72) Erfinder: **Brunner, Hans-Georg, Dr., Wannenstrasse 14, CH-4415 Lausen (CH)**
Erfinder: **Schurter, Rolf, Dr., Holzmattstrasse 45, CH-4102 Binningen (CH)**
Erfinder: **Szczepanski, Henry, Dr., Waldshuterstrasse 55, CH-4310 Rheinfelden (CH)**

EP 0 041 476 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Gegenstand der Erfindung sind neue Aryl-Phenyl-Acetylen-Verbindungen mit herbizider Wirkung, Verfahren zu deren Herstellung, sie enthaltende Mittel, sowie deren Verwendung zur selektiven Unkrautbekämpfung in verschiedenen Kulturen, wie z.B. Getreide, Mais und Reis. Die Erfindung betrifft auch als Zwischenprodukte dienende neue 1,1-Dialkyl-3-arylpropargylalkohole und deren Herstellung.

Den erfindungsgemässen neuen Aryl-Phenyl-Acetylen-Verbindungen und deren Ammoniumsalzen liegt die allgemeine Formel I

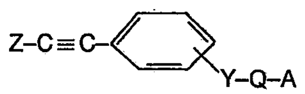 (I)

zugrunde, worin

A eine gegebenenfalls substituierte Aminogruppe,

Q eine $C_2$–$C_6$-Alkylenbrücke,

Y ein Sauerstoff- oder Schwefelatom, oder ein gegebenenfalls substituiertes Stickstoffatom und

Z einen gegebenenfalls substituierten Phenyl-, Naphthyl- oder heterocyclischen Rest bedeuten, wobei der Phenylrest mit dem Substituenten –Y–Q–A noch ein bis vier weitere Substituenten tragen kann, mit der Massgabe, dass Z nur dann für einen unsubstituierten Phenylkern steht, wenn der Phenylkern mit dem Substituenten –Y–Q–A einen weiteren Substituenten trägt oder der Substituent –Y–Q–A in der meta-Stellung zur Äthinylbrücke steht oder –Y–Q–A nicht $-O-CH_2-CH_2-N(C_2H_5)_2$ bedeutet.

Unter einer gegebenenfalls substituierten Aminogruppe sind primäre, sekundäre und tertiäre Aminogruppen zu verstehen.

Die Alkylenbrücke Q kann sowohl geradkettig als auch verzweigt sein.

Als Heterocyclen Z sind aromatische, Sauerstoff, Schwefel oder Stickstoff als Ringglied enthaltende, nicht kondensierte 5- bis 6gliedrige Ringe zu verstehen.

Bevorzugt sind Verbindungen der Formel Ia

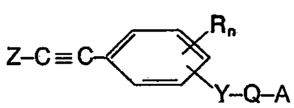 (Ia)

worin

A eine Aminogruppe der Formel

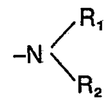 oder 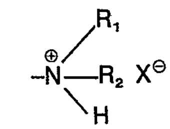 ,

$R_1$ und $R_2$ unabhängig voneinander jeweils Wasserstoff, $C_3$–$C_6$-Alkenyl, $C_3$–$C_6$-Alkinyl, $C_3$–$C_8$-Cycloalkyl oder gegebenenfalls durch Halogen, Hydroxy, $C_1$–$C_4$-Alkoxy, Alkoxycarbonyl mit höchstens 5 Kohlenstoffatomen, Cyan oder Carboxyl substituiertes $C_1$–$C_6$-Alkyl, oder

$R_1$ und $R_2$ zusammen mit dem sie tragenden Stickstoffatom auch einen 5- bis 6-gliedrigen gesättigten Heterocyclus mit insgesamt höchstens 2 Heteroatomen darstellen, der durch $C_1$–$C_3$-Alkyl substituiert sein kann,

$X^{\ominus}$ ein Anion,

Y Sauerstoff, Schwefel oder ein Radikal $-N\,R_4-$,

$R_4$ Wasserstoff, $C_3$–$C_6$-Alkenyl, $C_3$–$C_6$-Alkinyl, $C_3$–$C_8$-Cycloalkyl oder gegebenenfalls durch Hydroxy, $C_1$–$C_4$-Alkoxy, Alkoxycarbonyl mit höchstens 5-Kohlenstoffatomen, Cyan oder Carboxyl substituiertes $C_1$–$C_6$-Alkyl,

Q eine unverzweigte oder verzweigte $C_2$–$C_6$-Alkylenbrücke,

R Wasserstoff, Nitro, Cyan, Trifluormethyl, $C_1$–$C_4$-Alkoxy, $C_1$–$C_4$-Alkylthio, $-NR_5R_6$, $-CO-NR_7R_8$, $-COOR_9$, $-CO-SR_{10}$, Halogen, $-N_3$ oder gegebenenfalls durch $C_1$–$C_4$-Alkyl, Hydroxy, Cyan oder $-COOR_9$ substituiertes $C_1$–$C_4$-Alkyl,

n eine ganze Zahl von 1 bis 4,

$R_5$, $R_7$, $R_8$, $R_9$ und $R_{10}$ unabhängig voneinander jeweils Wasserstoff oder $C_1$–$C_6$-Alkyl, $C_3$–$C_8$-Alkenyl oder $C_3$–$C_8$-Alkinyl,

$R_6$ Wasserstoff, $C_1$–$C_6$-Alkyl, $C_3$–$C_8$-Alkenyl, $C_3$–$C_8$-Alkinyl, $-CO-R_{11}$, $-COO-R_{12}$ oder $-CO-NHR_{13}$ darstellen, wobei

$R_{11}$, $R_{12}$ und $R_{13}$ die gleiche Bedeutung wie $R_4$ haben und

Z für einen gegebenenfalls durch einen oder mehrere Reste, die dieselbe Bedeutung haben wie für R angegeben, oder die Formyl,

$-SO_2-NR_7R_8$, $-NH-NH_2$, $-NHOH$, $-SO-R_9$, $SO_2-R_9$, 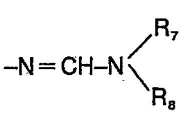 , $COO^{\ominus}\,M^{\oplus}$

oder gegebenenfalls durch Nitro, Cyano oder $-COOR_9$ substituiertes $C_2$–$C_6$-Alkenyl bedeuten, substituierten Phenyl-Naphthyl- oder heterocyclischen aromatischen Rest steht, der mindestens ein Sauerstoff-, Schwefel- oder Stickstoffatom enthält, wobei $M^{\oplus}$ für ein Natrium-, Kalium-, Calcium oder Magnesium-Kation steht, mit der Massgabe, dass Z dann ein substituierter Phenylrest, ein Naphthyl- oder heterocyclischer Rest sein muss, wenn –Y–Q–A in 4-Stellung zu Z–C≡C– steht, A Diäthylamino, R Wasserstoff, Q Äthylen und Y Sauerstoff ist.

Beispiele für Alkyl in den Definitionen von R und $R_1$ bis $R_{13}$, sind Methyl, Äthyl, n-Propyl, i-Propyl, die vier isomeren Butyl sowie die geradkettigen oder verzweigten Pentyl-, Hexyl-, Heptyl- oder Octylreste, vorzugsweise ist der Alkylrest jedoch gerad- und kurzkettig, insbesondere ist er durch Äthyl oder Methyl verkörpert.

Beispiele für Alkoxy in den Substituenten-Definitionen sind Methoxy, Äthoxy, n-Propyloxy, i-Propyloxy oder die vier isomeren Butyloxyreste, vorzugsweise jedoch Methoxy oder Äthoxy.

Beispiele für Alkylthio sind Methylthio, Äthylthio, n-Propylthio oder n-Butylthio, vorzugsweise jedoch Methylthio oder Äthylthio.

Beispiele für $C_3$–$C_8$-Cycloalkyl sind Cyclopropyl, Cyclobutyl, Cyclopentyl, Cyclohexyl, Cycloheptyl oder Cyclooctyl, vorzugsweise jedoch Cyclopropyl oder vor allem Cyclopentyl oder Cyclohexyl.

Beispiele für Anionen $X^\ominus$ sind die Anionen organischer und anorganischer Säuren wie Halogenwasserstoffsäuren, Phosphorsäure, Schwefelsäure, aliphatischer und aromatischer Sulfonsäuren, Fettsäuren sowie mehrwertiger organischer Säuren wie Oxalsäure, Malonsäure, Bernsteinsäure, Adipinsäure und Zitronensäure, vorzugsweise sind die Anionen jedoch Halogenidanionen, wie Chlorid oder Bromid.

Beispiele für Alkenylreste sind Allyl, die isomeren Butenyl-, Pentenyl-, Hexenyl-, Heptenyl- und Octenylreste, insbesondere aber Allyl.

Beispiele für Alkinylreste sind Propargyl, die isomeren Butinyl-, Pentinyl-, Hexinyl-, Heptinyl und Octininylreste, insbesondere aber Propargyl.

Beispiele für aromatische Heterocyclen sind Furyl, Thienyl, Triazolyl, Pyridyl, Oxazolyl, Thiazolyl, Thiadiazolyl, Pyrrolyl, Imidazolyl, Pyrazolyl, Pyrazinyl, Pyrimidinyl, Pyridazinyl, symmetrische und asymmetrische Triazinyl oder Oxadiazolylringe, vorzugsweise jedoch Furyl, Thienyl, Pyridyl, Pyrimidinyl, Pyrrolyl, Thiazolyl und Triazinyl.

Bevorzugte Halogensubstituenten sind Chlor, Brom und Jod.

Gesättigte Heterocyclen sind in der Regel Pyrrolidin, Imidazolidin, Pyrazolidin, Piperidin, Piperazin, Piperimidin, Morpholin und Thiomorpholin.

Die Alkylenbrücke Q wird durch geradkettige oder verzweigte Strukturen verkörpert wie z.B.: 1,2-Äthylen; 1,3-Propylen; 1,4-Butylen; 1,5-Pentylen; 1-Methyl-1,2-äthylen; 2-Methyl-1,2-äthylen; 1-Äthyl-1,2-äthylen; 2-Äthyl-1,2-äthylen; 1,2-Dimethyl-1,2-äthylen; 1-Methyl-1,3-propylen; 2-Methyl-1,3-propylen; 3-Methyl-1,3-propylen; 1-Äthyl-1,3-propylen; 2-Methyl-1,3-propylen, vorzugsweise besteht die direkte Kette zwischen dem Brückenatom Y und dem Stickstoffatom jedoch aus 2 oder 3 Kohlenstoffatomen.

Von den Verbindungen der Formel Ia sind diejenigen bevorzugt, in denen

a) der Rest –Y–Q–A in der 4-Position oder
b) in der 3-Position zum Aryläthinylrest steht,
c) die direkte Alkylenbrücke zwischen dem Brükkenatom Y und dem Stickstoffatom der Gruppe A aus 2 oder 3 Kohlenstoffatomen besteht,
d) der Arylrest Z gegebenenfalls substituiertes Phenyl; Furanyl- oder Thienyl oder
e) gegebenenfalls substituiertes Pyridyl, Pyrimidinyl, Pyrrolyl, Thiazolyl oder Trazinyl bedeutet,
f) die Reste $R_1$ und $R_2$ für Methyl oder Äthyl stehen und
g) das Brückenglied Y Sauerstoff ist.

Durch Kombination der obigen Merkmale ergeben sich die weiterhin bevorzugten Gruppen von Verbindungen der Formel Ia, in denen

aa) der Rest –Y–Q–A in der 4-Position zum Aryläthinylrest steht, die direkte Kette zwischen dem Brückenatom Y und dem Stickstoffatom der Gruppe A aus 2 bis 3 Kohlenstoffatomen besteht, der Arylrest Z gegebenenfalls substituiertes Phenyl, Furanyl oder Thienyl bedeutet, das Brückenglied Y Sauerstoff ist und die Reste $R_1$ und $R_2$ für Methyl oder Äthyl stehen,

bb) der Rest –Y–Q–A in der 3-Position zum Aryläthinylrest steht, die direkte Kette zwischen dem Brückenatom Y und dem Stickstoffatom der Gruppe A aus 2 bis 3 Kohlenstoffatomen besteht, der Arylrest Z gegebenenfalls substituiertes Phenyl, Furanyl oder Thienyl bedeutet, das Brückenglied Y Sauerstoff ist und die Reste $R_1$ und $R_2$ für Methyl oder Äthyl stehen,

cc) der Rest –Y–Q–A in der 4-Position zum Aryläthinylrest steht, die direkte Kette zwischen dem Brückenatom Y und dem Stickstoffatom der Gruppe A aus 2 bis 3 Kohlenstoffatomen besteht, die Arylreste Z gegebenenfalls substituiertes Pyridyl, Pyrimidinyl, Pyrrolyl, Thiazolyl oder Triazinyl bedeutet, das Brückenglied Y Sauerstoff ist und die Reste $R_1$ und $R_2$ für Methyl oder Äthyl stehen und

dd) der Rest –Y–Q–A in der 3-Position zum Aryläthinylrest steht, die direkte Kette zwischen dem Brückenatom Y und dem Stickstoffatom der Gruppe A aus 2 bis 3 Kohlenstoffatomen besteht, der Arylrest Z gegebenenfalls substituiertes Pyridyl, Pyrimidinyl, Pyrrolyl, Thiazolyl oder Triazinyl bedeutet, das Brückenglied Y Sauerstoff ist und die Reste $R_1$ und $R_2$ für Methyl oder Äthyl stehen.

Unter den Gruppen aa, bb, cc und dd von Verbindungen der Formel Ia sind jeweils die besonders bevorzugt, die am zentralen Phenylkern ausser dem Aryläthinylsubstituenten und der Gruppe –Y–Q–A höchstens einen weiteren Substituenten aus der Gruppe Halogen, $C_1$–$C_4$-Alkyl, Nitro oder Cyan tragen.

Als bevorzugte Einzelverbindungen sind zu nennen:

1-[4-(4-Methoxyphenyläthinyl)-phenoxy]-2-diäthyl-aminoäthan,
1-(3-Phenyläthinyl-phenoxy)-2-diäthylaminoäthan,
1-[4-(4-Fluorphenyläthinyl)-phenoxy]-2-diäthyl-aminoäthan,
1-(4-Phenyläthinyl-phenoxy)-2-äthylmethylamino-äthan,
1-[4-(2-Thienyläthinyl)-phenoxy]-2-diäthylaminoäthan,
1-Methyl-1-(3-phenyläthinyl-phenoxy)-2-dimethyl-aminoäthan und
2-Methyl-1-(3-phenyläthinyl-phenoxy)-2-dimethyl-aminoäthan.

Die Aryl-Phenyl-Acetylen-Verbindungen der Formel I sind neu und werden nach folgenden

Methoden hergestellt, wobei die Formeln IV und X im Schema A als Unterformeln von I zu verstehen sind:

Schema A:

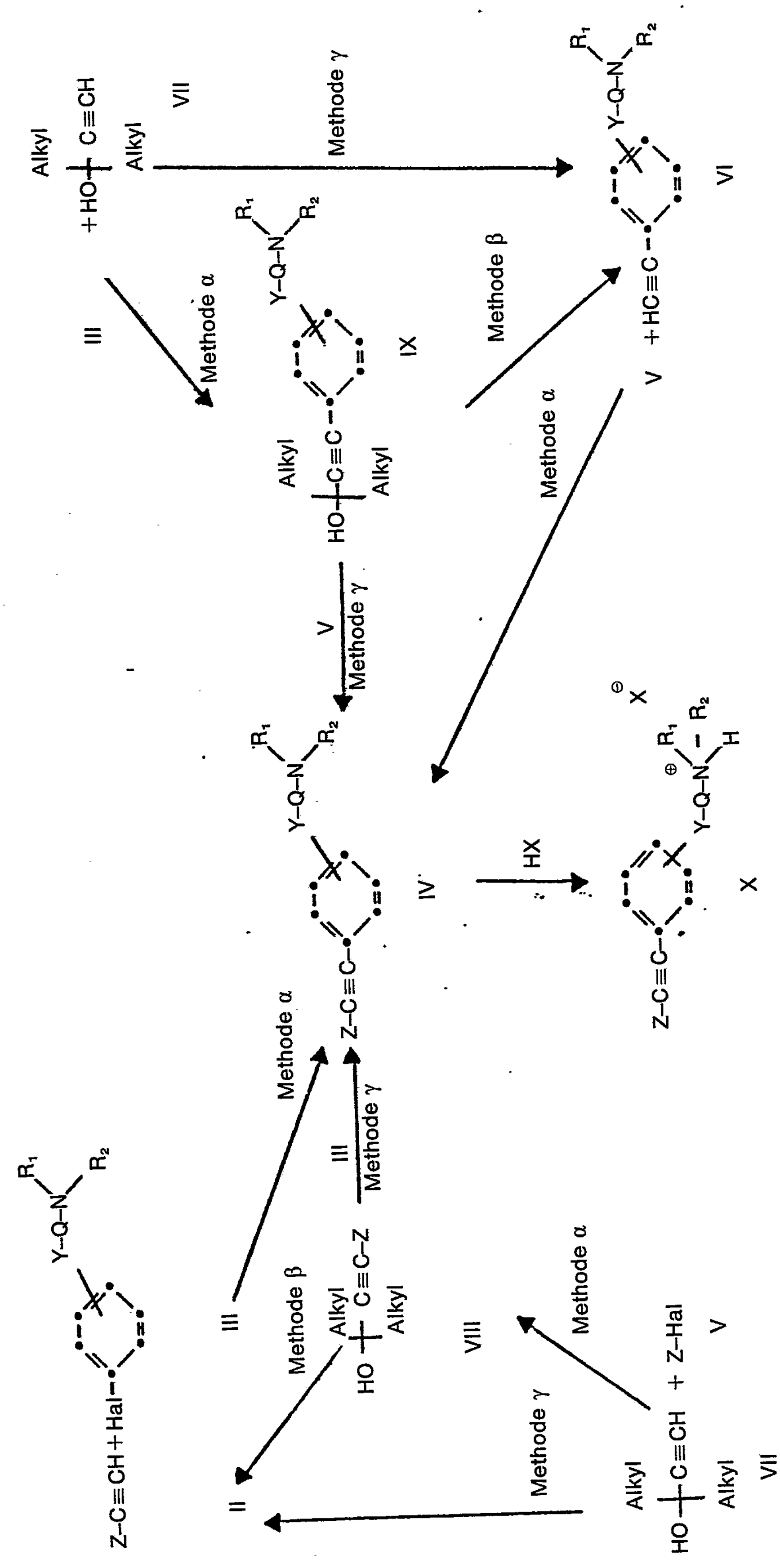

Im Schema A haben $R_1$, $R_2$, Q, X, Y und Z die unter Formel I und Ia angegebene Bedeutung. Unter Halogen ist Chlor, insbesondere aber Brom und Jod zu verstehen.

Methode α ist ein Verfahren, das es erlaubt, unter Verwendung von Metallkatalysatoren, halogenierte aromatische Reste, wie in den Formeln III oder V, mit endständigen Acetylengruppen, wie in den Formeln II, VI und VII, unter milden Reaktionsbedingungen in Gegenwart eines säurebindenden Mittels zu binden. Reaktionen dieser Art sind in folgenden Literaturstellen beschrieben: K. Sonogashira, Y. Tohda und N. Hagihara, Tetrahedron Lett., 50, 4467 (1975); L. Cassar, J. Organomet. Chem., 93, 253 (1975) und H. A. Dieck und F. R. Heck, J. Organomet. Chem., 93, 259 (1975).

Diese Reaktion findet vorteilhafterweise in gegenüber den Reaktionspartnern inerten, organischen Lösungsmitteln statt. Als solche kommen viele protische wie auch aprotische Lösungsmittel in Frage, z. B. Alkanole, Ketone, Äther, Kohlenwasserstoffe, halogenierte Kohlenwasserstoffe, aromatische Lösungsmittel oder auch z. B. Dimethylformamid, Dimethylsulfoxid, Acetonitril oder tertiäre Amine.

Als säurebindendes Mittel kann, da bei der Umsetzung Halogenwasserstoff abgespalten wird, eine Base eingesetzt werden. Dafür kommen starke anorganische Basen wie KOH, NaOH in Frage, aber auch organische wie beispielsweise Triäthylamin, Diäthylamin, Pyridin, Alkoholate etc. Bei den Reaktionen werden 1–5 Äquivalente dieser Basen eingesetzt.

Als Metallkatalysatoren werden vorzugsweise Palladiumsalze oder -komplexe verwendet, insbesondere Palladiumchlorid $PdCl_2$, Palladiumacetat $Pd(OCOCH_3)_2$ oder der Palladiumdichlor-bis-(triphenylphosphin)-Komplex $Pd\ Cl_2[P(C_6H_5)_3]_2$, meist unter Zusatz eines Kupfer-(I)-Salzes, insbesondere von Kupfer-(I)-jodid. Die Katalysatoren werden als solche oder aufgezogen auf einem Träger wie z. B. Kohlepulver, Aluminiumoxid etc., verwendet.

Die Reaktionstemperaturen liegen in der Regel zwischen 0° und 200 °C, vorwiegend jedoch zwischen Raumtemperatur und der Siedetemperatur des Reaktionsgemisches. Da die Reaktionen oft leicht exotherm verlaufen, wird zur Steigerung der Reaktionsgeschwindigkeit vorteilhaft während kurzer Zeit die Temperatur im Reaktionsgefäss erhöht.

Die Reaktionszeiten liegen im allgemeinen zwischen 0,5 und 48 Stunden.

Die Methode β ermöglicht es, in Gegenwart einer starken Base wie NaOH, KOH oder Alkoholat aus einem tertiären Äthinyläthanol, wie in den Formeln VII, VIII und IX, welches als geschützte endständige Acetylengruppe aufgefasst werden kann, unter Abspaltung der Ketoschutzgruppe das Acetylen, wie in den Formeln II, VI und IX, freizusetzen. Die als Nebenprodukte entstehenden Ketone können aus dem Reaktionsgemisch während der Reaktion destillativ entfernt werden. Reaktionen dieser Art sind in der DOS 2 905 507 und im US-Patent 4 128 588 beschrieben.

Diese Reaktion wird vorteilhafterweise in gegenüber den Reaktionspartnern inerten, organischen Lösungsmitteln wie Alkoholen, Äthern, Ketonen, Kohlenwasserstoffen, Halogenkohlenwasserstoffen, aromatischen Lösungsmitteln oder auch Dimethylformamid, Dimethylsulfoxid oder Acetonitril durchgeführt.

Die Reaktionstemperatur liegt auch hier vorzugsweise zwischen Raumtemperatur und dem Siedepunkt des Reaktionsgemisches.

Die Reaktionszeit beträgt im allgemeinen zwischen 0,5 und 12 Stunden.

Die Methode γ besteht aus einer Kombination der Methoden α und β, wobei das nach der Methode α umzusetzende Acetylen in situ durch Einwirkung einer starken Base auf ein geschütztes Acetylen der Formeln VII, VIII und IX hergestellt wird.

Die Reaktionsbedingungen sind mit denen der Methode α identisch.

Zwingend erforderlich ist jedoch der Zusatz einer starken Base wie NaOH, KOH oder das Alkalisalz eines Alkohols.

Nach dem erfindungsgemässen Verfahren werden die Verbindungen der Formel IV

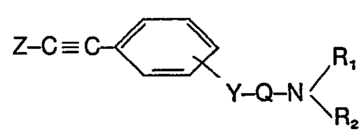
(IV)

erhalten, indem man entweder eine Äthinylverbindung der Formel II

$$Z\text{–}C\equiv CH \qquad (II)$$

in Gegenwart eines säurebindenden Mittels und eines Metallkatalysators gegebenenfalls unter einer Inertgasatmosphäre mit einem Phenylhalogenid der Formel III

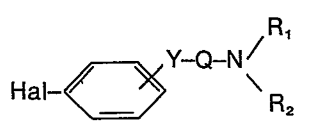
(III)

umsetzt oder indem man ein aromatisches Halogenid der Formel V

$$Z\text{–}Hal \qquad (V)$$

unter den gleichen Reaktionsbedingungen mit einem Phenylacetylen der Formel VI

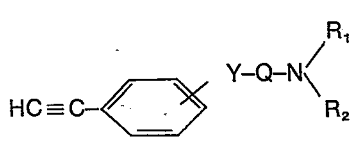
(VI)

worin $R_1$, $R_2$, Y, Q und Z die unter Formel I und Ia gegebene Bedeutung haben und Hal Brom oder Jod ist, umsetzt.

Nach einem weiteren erfindungsgemässen Verfahren werden die Verbindungen der Formel IV

erhalten, indem man ein aromatisches Halogenid der Formel V

Z–Hal (V)

in Gegenwart eines säurebindenden Mittels und eines Metallkatalysators gegebenenfalls unter einer Inertgasatmosphäre mit einem Propargylalkohol der Formel VII

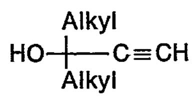 (VII)

umsetzt und die erhaltene Äthinylverbindung der Formel VIII

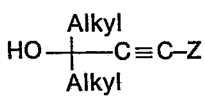 (VIII)

in Gegenwart einer starken Base und eines Metallkatalysators gegebenenfalls unter einer Inertgasatmosphäre mit einem Phenylhalogenid der Formel III

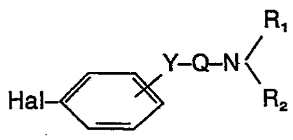 (III)

umsetzt oder indem man das Phenylhalogenid der Formel III unter den obigen Bedingungen zuerst mit dem Propargylalkohol der Formel VII umsetzt und dann die entstandene Äthinylverbindung der Formel IX

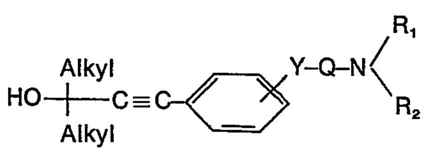 (IX)

unter den obigen Bedingungen mit dem aromatischen Halogenid der Formel V, worin $R_1$, $R_2$, Q, Y und Z die unter Formel IV gegebene Bedeutung haben, Hal Brom oder Jod ist und Alkyl ein $C_1$–$C_4$-Alkylrest ist, umsetzt.

Die teilweise neuen und speziell als Zwischenprodukte für die Verbindungen der Formel I entwickelten 1,1-Dialkyl-3-arylpropargylalkohole der Formeln VIII und IX werden analog zum ersten Reaktionsschritt des vorgenannten Verfahrens hergestellt, indem man Halogenide der Formeln V resp. VI mit einem Propargylalkohol der Formel VII umsetzt.

Die Verbindungen der Formel IX sind neu und bilden ebenfalls einen Gegenstand der vorliegenden Erfindung.

Nach einem weiteren Verfahren erhält man die erfindungsgemässen Verbindungen der Formel X

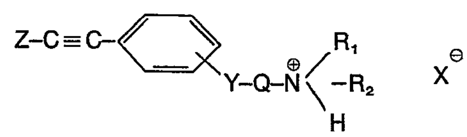 (X)

indem man die Verbindungen der Formel IV mit einer Säure HX umsetzt, worin $R_1$, $R_2$, Q, X, Y und Z die unter Formel I und Ia gegebene Bedeutung haben.

Die Ausgangsmaterialien der Formeln II, III, V, VI, VII und VIII sind bekannt oder leicht herstellbar und/oder im Handel erhältlich.

Die neuen Wirkstoffe der Formel I sind stabile Verbindungen.

Die Verbindungen der Unterformel IV sind in den üblichen organischen Lösungsmitteln relativ gut und in Wasser schlecht löslich. Sie können durch Zugeben von Wasser zur Reaktionslösung leicht ausgefällt werden. Ihre Formulierung als flüssige herbizide Mittel gelingt mit Hilfe üblicher Lösungsvermittler und/oder Dispergiermittel.

Die Verbindungen der Unterformel X sind in Wasser gut, jedoch selbst in polaren organischen Lösungsmitteln wie Dimethylsulfoxid, Dimethylformamid oder Acetonitril relativ schlecht löslich.

Die herbizide Wirksamkeit von 1-(4-Phenyläthinyl-phenoxy)-2-diäthylaminoäthan ist vor kurzem entdeckt und zum ersten Mal in Phytochemistry, 19, 61 (1980) beschrieben worden.

Die erfindungsgemässen Aryl-Phenyl-Acetylen-Verbindungen der Formel I beeinflussen das Pflanzenwachstum und zeigen bei post-emergenter Applikation ausgezeichnete selektiv-herbizide Wirkung gegen schwer bekämpfbare breitblättrige Unkräuter wie Galium, Veronica, Viola, aber auch gegen Sinapis. Chrysanthemum etc. in Mais und Reis, vorwiegend aber in Getreide-Kulturen. Besonders mehrjährige Unkräuter werden durch die Translokation der Wirkstoffe der Formel I wirksam bekämpft.

Unter Translokation ist der Transport eines Wirkstoffes innerhalb der Pflanze zu verstehen. Dabei kann der Wirkstoff von den Blättern in die Wurzel und umgekehrt transloziert werden und dort seine Wirkung entfalten. Die Wirkstoffe der Formel I sind translozierbar, d.h. mit ihnen werden z.B. durch Blattapplikation Unkräuter bis in die Wurzel hinein geschädigt.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder

flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$–$C_{22}$), wie z. B die Na- oder K-Salze der Öl- oder Stearinsäure, oder von natürlichen Fettsäuregemischen, die z. B. aus Kokosnuss- oder Talgöl gewonnen werden können, genannt. Ferner sind auch die Fettsäure-methyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit 8–22 C-Atomen. Alkylarylsulfonate sind z. B. die Na-, Ca- oder Triäthanolaminsalze der Doedecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxid-Adduktes in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylulfate vor, z. B. das Stearyltrimethylammoniumchlorid oder das Benzyldi(2-chloräthyl)äthylammoniumbromid.

Die in der Formulierungstechnik gebräuchlichen Tenside sind u. a. in folgenden Publikationen beschrieben:

«Mc Cutcheon's Detergents and Emulsifiers Annual» MC Publishing Corp., Ridgewood, New Jersey, 1979. Sisley and Wood, «Encyclopedia of Surface Active Agents», Chemical Publishing Co., Inc. New York, 1964.

Die pestiziden Zubereitungen enthalten in der Regel 0,1 bis 99%, insbesondere 0,1 bis 95%,

Wirkstoff der Formel I, 1 bis 99% eines festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent)

| | |
|---|---|
| Lösungen | |
| Aktiver Wirkstoff: | 5 bis 95%, vorzugsweise 10 bis 80% |
| Lösungsmittel: | 95 bis 5%, vorzugsweise 90 bis 0% |
| oberflächenaktives Mittel: | 1 bis 30%, vorzugsweise 2 bis 20%. |
| Emulgierbare Konzentrate: | |
| Aktiver Wirkstoff: | 10 bis 50%, bevorzugt 10 bis 40% |
| oberflächenaktives Mittel: | 5 bis 30%, vorzugsweise 10 bis 20% |
| flüssiges Trägermittel: | 20 bis 95%, vorzugsweise 40 bis 80%. |
| Stäube | |
| Aktiver Wirkstoff: | 0,5 bis 10%, vorzugsweise 2 bis 8% |
| festes Trägermittel: | 99,5 bis 90%, vorzugsweise 98 bis 92%. |
| Suspension-Konzentrate | |
| Aktiver Wirkstoff: | 5 bis 75%, vorzugsweise 10 bis 50% |
| Wasser: | 94 bis 25%, vorzugsweise 90 bis 30% |
| oberflächenaktives Mittel: | 1 bis 40%, vorzugsweise 2 bis 30%. |
| Benetzbare Pulver | |
| Aktiver Wirkstoff: | 5 bis 90%, vorzugsweise 10 bis 80% und insbesondere 20 bis 60% |
| oberflächenaktives Mittel: | 0,5 bis 20%, vorzugsweise 1 bis 15% |
| festes Trägermittel: | 5 bis 90%, vorzugsweise 30 bis 70%. |
| Granulate | |
| Aktiver Wirkstoff: | 0,5 bis 30%, vorzugsweise 3 bis 15% |
| festes Trägermittel: | 99,5 bis 70%, vorzugsweise 97 bis 85%. |

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,1 bis 10 kg AS/ha, vorzugsweise 0,25 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

Herstellungsbeispiele

Beispiel 1:
1-(3-Phenyläthinyl-phenoxy)-2-diäthylaminoäthan.

(Verb. 1)

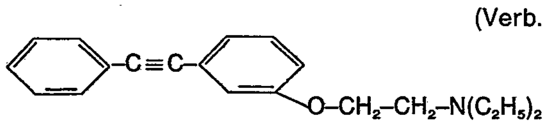

Einer Lösung von 15 g (0,06 Mol) 1-(3-Bromphenoxy)-2-diäthylaminoäthan und 6,12 g (0,06 Mol) Phenylacetylen in 150 ml Triäthylamin werden unter Stickstoff 420 mg Palladiumkomplex $PdCl_2[P(C_6H_5)_3]_2$ und 228 mg Kupfer-(I)-jodid CuJ zugesetzt. Anschliessend wird diese Reaktionsmischung für 24 Stunden am Rückfluss gekocht. Nach Einengen und Versetzen mit Wasser wird zweimal mit 200 ml Äther extrahiert. Die vereinigten Ätherphasen werden viermal mit Wasser gewaschen, getrocknet und eingedampft. Das erhaltene Rohprodukt wird mit Chloroform an Silikagel chromatographiert und anschliessend destilliert. Man erhält 13,1 g (75%) 1-(3-Phenyläthinyl-phenoxy)-2-diäthylaminoäthan vom Siedebereich 150–160°/0,02 mb mit einem Brechungsindex $n_D^{25}$: 1,5858.

Beispiel 2:
1-(2,5-Dichlor-4-phenyläthinyl-phenoxy)-2-diäthylaminoäthan.

(Verb. 407)

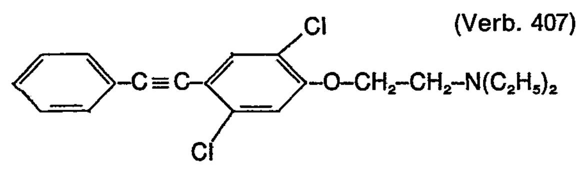

Einer Lösung von 19,4 g (0,05 Mol) 1-(2,5-Dichlor-4-jod-phenoxy)-2-diäthylaminoäthan und 5,2 g (0,053 Mol) Phenylacetylen in 250 ml Triäthylamin werden unter Stickstoff 200 mg Palladiumkomplex $PdCl_2[P(C_6H_5)_3]_2$ und 100 mg Kupfer-(I)-jodid zugesetzt. Nach Abklingen der schwach exothermen Reaktion wird das Reaktionsgemisch für 15 Stunden bei Raumtemperatur gerührt. Nach Einengen und Versetzen mit Wasser wird zweimal mit 200 ml Äther extrahiert. Die vereinigten Ätherphase werden viermal mit Wasser gewaschen, getrocknet und eingedampft. Das erhaltene Rohprodukt wird mit Chloroform an Silikagel chromatographiert. Man erhält 14,3 g (79%) 1-(2,5-Dichlor-4-phenyläthinylphenoxy)-2-diäthylaminäthan mit einem Brechungsindex $n_D^{25}$: 1,6173.

Beispiel 3:
1-[4-(4-Methoxyphenyläthinyl)-phenoxy]-2-diäthylaminoäthan.

(Verb. 433)

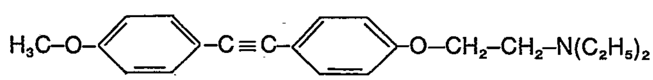

Einer Lösung von 15 g (0,06 Mol) 1-(4-Bromphenoxy)-2-diäthylaminoäthan und 8,2 g (0,062 Mol) 4-Methoxyphenylacetylen in 150 ml Triäthylamin werden unter Stickstoff 420 mg Palladiumkomplex $PdCl_2[P(C_6H_5)_3]_2$ und 228 mg Kupfer-(I)-jodid CuJ zugesetzt. Anschliessend wird die Reaktionsmischung für 24 Stunden am Rückfluss gekocht. Nach Einengen und Versetzen mit Wasser wird zweimal mit 200 ml Äther extrahiert. Die vereinigten Ätherphasen werden dreimal mit Wasser gewaschen, getrocknet und eingedampft. Das erhaltene Rohprodukt wird mit Chloroform an Silikagel chromatographiert. Man erhält so 15,5 g (80%) 1-[4-(4-Methoxyphenyläthinyl)-phenoxy]-2-diäthylaminoäthan vom Schmelzpunkt 59–60 °C.

Beispiel 4:
1-[4-(2-Thienyläthinyl)-phenoxy]-2-diäthylaminoäthan.

(Verb. 471)

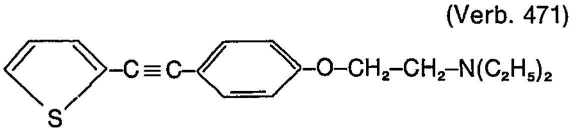

a) Zu 142 g (0,45 Mol) 1-(4'-Jodphenoxy)-2-diäthylaminoäthan, 1,2 l Triäthylamin und 37,8 g (0,45 Mol) 3-Methyl-1-butin-3-ol werden unter Stickstoff 3,4 g Palladiumkomplex $PdCl_2[P(C_6H_5)_3]_2$ und 1,9 g Kupfer-(I)-jodid zugesetzt. Nach Abklingen der exothermen Reaktion wird das Reaktionsgemisch für 15 Stunden bei Raumtemperatur gerührt. Das Reaktionsgemisch wird filtriert und die Lösung im Vakuum eingedampft. Der erhaltene ölige Rückstand kristallisiert sofort.

Das Produkt wird bei 40 °C am Vakuum getrocknet.

Man erhält so 123 g (99%) 1-(3-Hydroxy-3-methyl-1-butin-1-yl)-4-(2-diäthylaminoäthoxy)-benzol vom Schmelzpunkt 64 °C.

b) Einer Lösung von 11,07 g (0,04 mol) 1-(3-Hydroxy-3-methyl-1-butin-1-yl)-4-(2-diäthylaminoäthoxy)-benzol und 8,4 g (0,04 Mol) 2-Jodthiophen in 135 ml Triäthylamin werden unter Stickstoff 4,95 g pulverisiertes Kaliumhydroxid, 310 mg Palladiumkomplex $PdCl_2[P(C_6H_5)_3]_2$ und 180 mg Kupfer-(I)-jodid zugesetzt. Anschliessend wird das Reaktionsgemisch 20 Stunden bei Siedetemperatur gerührt. Nach Aufnehmen des Reaktionsgemisches mit 250 ml Äther, dreimal Waschen mit 100 ml Wasser und einmal mit gesättigter Kochsalzlösung, Trocknen mit Magnesiumsulfat und Eindampfen wird der Rückstand mit 96% Äther/4% Methanol (gesättigt mit $NH_3$)-Gemisch an Kieselgel chromatographiert. Man erhält so 9,0 g (75%) 1-[4-(2-Thienyläthinyl)-phenoxy]-2-diäthylaminoäthan als farbloses Öl. $n_D^{30}$ 1,6170.

Beispiel 5:
1-[4-(4-Fluorphenyläthinyl)-phenoxy]-2-diäthylaminoäthan.

(Verb. 416)

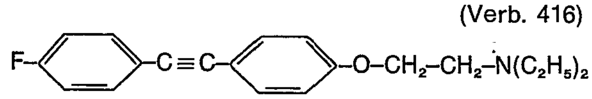

a) Einer Lösung von 35,2 g (0,19 mol) 4-Bromfluorbenzol und 25,2 g (0,3 Mol) 3-Hydroxy-3-methyl-1-butin in 250 ml Triäthylamin werden unter Stickstoff 1,0 g Palladiumkomplex $PdCl_2[P(C_6H_5)_3]_2$ und 0,2 g Kupfer-(I)-jodid zugesetzt. Anschliessend wird das Reaktionsgemisch für 24 Stunden bei 70° gerührt. Nach Abfiltrieren des Triäthylammoniumbromids und Abdampfen des überschüssigen Triäthylamins wird der Rückstand mit 200 ml Äther aufgenommen und je zweimal mit 100 ml 5%-iger wässriger HCl-Lösung und 100 ml Wasser gewaschen, getrocknet, mit Aktivkohle behandelt und eingedampft. Der Rückstand wird mit 1 : 1-Äther-Hexan-Gemisch an Kieselgel chromatographiert. Man erhält 26,4 g (78%) 1-Fluor-4-(3-hydroxy-3-methyl-1-butin-1-yl)-benzol vom Schmelzpunkt 124°.

b) Einer Lösung von 25,2 g (0,1 Mol) 1-(4-Bromphenoxy)-2-diäthylaminoäthan und 17,8 g (0,1 Mol) 1-Fluor-4-(3-hydroxy-3-methyl-1-butin-1-yl)-benzol in 250 ml Triäthylamin werden unter Stickstoff 11,2 g pulverisiertes Kaliumhydroxid, 0,7 g Palladiumkomplex $PdCl_2[P(C_6H_5)_3]_2$ und 0,13 g Kupfer-(I)-jodid zugesetzt. Anschliessend wird das Reaktionsgemisch für 14 Stunden bei 80° gerührt. Nach Aufnehmen des Reaktionsgemisches mit 250 ml Äther, Waschen mit je zweimal 100 ml Wasser und 100 ml 10%-iger wässriger $K_2CO_3$-Lösung, Trocknen, Behandeln mit Aktivkohle und

Eindampfen werden durch Vakuumdestillation 9,5 g (33%) 1-[4-(4-Fluorphenyläthinyl)-phenoxy]-2-diäthylaminoäthan mit einem Siedepunkt von 156–162°/0,08 mb erhalten.

Die aus den Beispielen erhaltenen und in analoger Weise herzustellende Verbindung sind in folgender Tabelle aufgeführt:

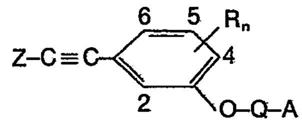

| Nr. | Z | $R_n$ | Q | A | phys. Daten |
|---|---|---|---|---|---|
| 1 | $C_6H_5-$ | H | $-(CH_2)_2-$ | $-N(C_2H_5)_2$ | Kp: 150–160°/ 0,02mb $n_D^{25}$: 1,5858 |
| 2 | $C_6H_5-$ | H | $-(CH_2)_2-$ | 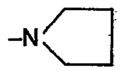 | |
| 3 | $C_6H_5-$ | H | $-(CH_2)_2-$ | 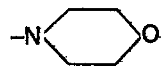 | |
| 4 | $C_6H_5-$ | H | $-(CH_2)_3-$ | $-N(C_2H_5)_2$ | |
| 5 | $C_6H_5-$ | H | $-(CH_2)_2-$ | $-NHC_2H_5$ | |
| 6 | $C_6H_5-$ | H | $-(CH_2)_2-$ | $-N(C_2H_5)_2 \cdot HCl$ | |
| 7 | $C_6H_5-$ | 4–Cl | $-(CH_2)_2-$ | $-N(C_2H_5)_2$ | |
| 8 | $C_6H_5-$ | $4-NO_2$ | $-(CH_2)_2-$ | $-N(C_2H_5)_2$ | |
| 9 | $C_6H_5-$ | 4–CN | $-(CH_2)_2-$ | $-N(C_2H_5)_2$ | |
| 10 | $C_6H_5-$ | $4-CH_3$ | $-(CH_2)_2-$ | $-N(C_2H_5)_2$ | |
| 11 | $C_6H_5-$ | $4-C_2H_5$ | $-(CH_2)_2-$ | $-N(C_2H_5)_2$ | |
| 12 | $C_6H_5-$ | $4-OCH_3$ | $-(CH_2)_2-$ | $-N(C_2H_5)_2$ | |
| 13 | $C_6H_5-$ | $4-CH_3$, $6-CH_3$ | $-(CH_2)_2-$ | $-N(C_2H_5)_2$ | |
| 14 | $C_6H_5-$ | 4–Cl, $6-CH_3$ | $-(CH_2)_2-$ | $-N(C_2H_5)_2$ | |
| 15 | $C_6H_5-$ | $2-CH_3$, $4-CH_3$ | $-(CH_2)_2-$ | $-N(C_2H_5)_2$ | |
| 16 | $C_6H_5-$ | $2-CH_3$, $6-CH_3$ | $-(CH_2)_2-$ | $-N(C_2H_5)_2$ | |
| 17 | $C_6H_5-$ | 4–Cl | $-(CH_2)_3-$ | $-N(C_2H_5)_2$ | |
| 18 | $C_6H_5-$ | 4–Cl | $-(CH_2)_3-$ | 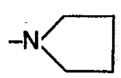 | |
| 19 | $C_6H_5-$ | $4-NO_2$ | $-(CH_2)_3-$ | $-N(C_2H_5)_2$ | |
| 20 | $C_6H_5-$ | $4-CH_3$ | $-(CH_2)_3-$ | $-N(C_2H_5)_2$ | |
| 21 | $C_6H_5-$ | 4–Cl | $-(CH_2)_3-$ | $-N(CH_3)_2$ | |
| 22 | $C_6H_5-$ | $4-OCH_3$ | $-(CH_2)_3-$ | $-N(CH_3)_2$ | |
| 23 | $4-Cl-C_6H_4-$ | H | $-(CH_2)_2-$ | $-N(C_2H_5)_2$ | |

| Nr. | Z | $R_n$ | Q | A | phys. Daten |
|---|---|---|---|---|---|
| 24 | $4-Cl-C_6H_4-$ | 4-Cl | $-(CH_2)_2-$ | $-N(C_2H_5)_2$ | |
| 25 | $4-CF_3-C_6H_4-$ | H | $-(CH_2)_2-$ | $-N(C_2H_5)_2$ | |
| 26 | $4-CF_3-C_6H_4-$ | H | $-(CH_2)_3-$ | $-N(C_2H_5)_2$ | |
| 27 | $4-CF_3-C_6H_4-$ | $4-NO_2$ | $-(CH_2)_2-$ | $-N(C_2H_5)_2$ | |
| 28 | $4-CF_3-2-Cl-C_6H_3-$ | H | $-(CH_2)_2-$ | $-N(C_2H_5)_2$ | |
| 29 | $4-CF_3-2-Cl-C_6H_3-$ | $4-OCH_3$ | $-(CH_2)_2-$ | $-N(C_2H_5)_2$ | |
| 30 | $4-CF_3-2-Cl-C_6H_3-$ | H | $-(CH_2)_3-$ | $-N(C_2H_5)_2$ | |
| 31 | $3-CH_3-5-CH_3-C_6H_3$ | H | $-(CH_2)_2-$ | $-N(C_2H_5)_2$ | |
| 32 | $3-CH_3-5-CH_3-C_6H_3$ | 4-Cl | $-(CH_2)_2-$ | $-N(C_2H_5)_2$ | |
| 33 | $3-CH_3-5-CH_3-C_6H_3$ | H | $-(CH_2)_3-$ | $-N(C_2H_5)_2$ | |
| 34 | $4-iC_3H_7-C_6H_4-$ | H | $-(CH_2)_3-$ | $-N(C_2H_5)_2$ | |
| 35 | $4-iC_3H_7-C_6H_4-$ | H | $-(CH_2)_2-$ | $-N(C_2H_5)_2$ | |
| 36 | $4-CN-C_6H_4-$ | H | $-(CH_2)_2-$ | $-N(C_2H_5)_2$ | |
| 37 | $4-CN-C_6H_4-$ | $4-CH_3$ | $-(CH_2)_2-$ | $-N(C_2H_5)_2$ | |
| 38 | $4-NO_2-C_6H_4-$ | H | $-(CH_2)_2-$ | $-N(C_2H_5)_2$ | |
| 39 | $4-NO_2-C_6H_4-$ | H | $-(CH_2)_3-$ | $-N(C_2H_5)_2$ | |
| 40 | $4-NO_2-C_6H_4-$ | $4-C_2H_5$ | $-(CH_2)_2-$ | $-N(C_2H_5)_2$ | |
| 41 | $2-NO_2-4-CL-C_6H_3-$ | H | $-(CH_2)_2-$ | $-N(C_2H_5)_2$ | |
| 42 | $2-NO_2-4-Cl-C_6H_3-$ | 4-Cl | $-(CH_2)_2-$ | $-N(C_2H_5)_2$ | |
| 43 | $2-C_2H_5-6-CH_3-C_6H_3-$ | H | $-(CH_2)_2-$ | $-N(C_2H_5)_2$ | |
| 44 | $2-C_2H_5-6-CH_3-C_6H_3-$ | H | $-(CH_2)_2-$ | $-N(OCH_3)CH_3$ | |
| 45 | $2-C_2H_5-6-CH_3-C_6H_3-$ | $4-NO_2$ | $-(CH_2)_2-$ | $-N(C_2H_5)_2$ | |
| 46 | $4-OCH_3-C_6H_4-$ | H | $-(CH_2)_2-$ | $-N(C_2H_5)_2$ | $n_D^{25}$: 1,6092 |
| 47 | $4-OCH_3-C_6H_4-$ | 4-Cl | $-(CH_2)_2-$ | $-N(C_2H_5)_2$ | |
| 48 | $4-SC_2H_5-C_6H_4-$ | H | $-(CH_2)_2-$ | $-N(C_2H_5)_2$ | |
| 49 | $4-F-C_6H_4-$ | H | $-(CH_2)_2-$ | $-N(C_2H_5)_2$ | $n_D^{25}$: 1,5818 |
| 50 | $3-CF_3-C_6H_4-$ | H | $-(CH_2)_2-$ | $-N(C_2H_5)_2$ | |
| 51 | $3-CF_3-C_6H_4-$ | H | $-(CH_2)_3-$ | $-N(C_2H_5)_2$ | |
| 52 | $3-CF_3-C_6H_4-$ | H | $-(CH_2)_3-$ | $-N(C_3)_2$ | |
| 53 | $3-CF_3-5-CF_3-C_6H_3-$ | H | $-(CH_2)_2-$ | $-N(C_2H_5)_2$ | |
| 54 | $3-CF_3-5-CF_3-C_6H_3-$ | H | $-(CH_2)_2-$ | $-N(OCH_3)CH_3$ | |
| 55 | $3-CF_3-5-CF_3-C_6H_3-$ | H | $-(CH_2)_2-$ | $-N(OCH_3)CH_3$ | |

| Nr. | Z | $R_n$ | Q | A | phys. Daten |
|---|---|---|---|---|---|
| 56 | $3–CN–C_6H_4–$ | H | $–(CH_3)_2–$ | $–N(C_2H_5)_2$ | |
| 57 | $3–CN–C_6H_4–$ | H | $–(CH_2)_3–$ | $–N(C_2H_5)_2$ | |
| 58 | $3–CN–C_6H_4–$ | 4–Cl | $–(CH_2)_2–$ | $–N(C_2H_5)_2$ | |
| 59 | $3–CN–C_6H_4–$ | H | $–(CH_2)_3–$ | $–N(C_3)_2$ | |
| 60 | $3–COOCH_3–C_6H_4–$ | H | $–(CH_2)_2–$ | $–N(CH_3)_2$ | |
| 61 | $3–COOCH_3–C_6H_4–$ | H | $–(CH_2)_3–$ | $–N(CH_3)_2$ | |
| 62 | $3–COOCH_3–C_6H_4–$ | H | $–(CH_2)_3–$ | $–N(OCH_3)CH_3$ | |
| 63 | $3–COOCH_3–C_6H_4–$ | $4–NO_2$ | $–(CH_2)_2–$ | $–N(C_2H_5)_2$ | |
| 64 | $3–OCH_3–C_6H_4–$ | H | $–(CH_2)_2–$ | $–N(C_2H_5)_2$ | |
| 65 | $3–OCH_3–C_6H_4–$ | H | $–(CH_2)_3–$ | $–N(C_2H_5)_2$ | |
| 66 | $3–OCH_3–C_6H_4–$ | 4–CN | $–(CH_2)_3–$ | $–N(C_2H_5)_2$ | |
| 67 | $3–OCH_3–C_6H_4–$ | 4–CN | $–(CH_2)_2–$ | $–N(C_2H_5)_2$ | |
| 68 | $3–CON(CH_3)_2–C_6H_4–$ | H | $–(CH_2)_2–$ | $–N(C_2H_5)_2$ | |
| 69 | $3–CON(CH_3)_2–C_6H_4–$ | H | $–(CH_2)_3–$ | $–N(C_2H_5)_2$ | |
| 70 | $3–CON(CH_3)_2–C_6H_4–$ | 4–Cl | $–(CH_2)_2–$ | $–N(C_2H_5)_2$ | |
| 71 | $3–CON(CH_3)_2–C_6H_4–$ | H | $–(CH_2)_2–$ | $–N(nC_4H_9)_2$ | |
| 72 | $3–COSC_2H_5–C_6H_4–$ | H | $–(CH_2)_2–$ | $–N(C_2H_5)_2$ | |
| 73 | $3–COSC_2H_5–C_6H_4–$ | H | $–(CH_2)_3–$ | $–N(C_2H_5)_2$ | |
| 74 | $3–COSC_2H_5–C_6H_4–$ | $4–NO_2$ | $–(CH_2)_2–$ | $–N(C_2H_5)_2$ | |
| 75 | $3–COSC_2H_5–C_6H_4–$ | $4–NO_2$ | $–(CH_2)_2–$ | $–N(nC_4H_9)_2$ | |
| 76 | 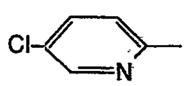 | H | $–(CH_2)_2–$ | $–N(C_2H_5)_2$ | |
| 77 | 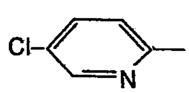 | H | $–(CH_2)_3–$ | $–N(C_2H_5)_2$ | |
| 78 | 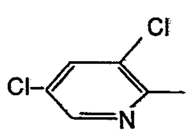 | H | $–(CH_2)_2–$ | $–N(C_2H_5)_2$ | |
| 79 | 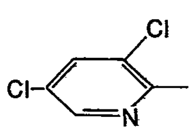 | $4–NO_2$ | $–(CH_2)_2–$ | $–N(C_2H_5)_2$ | |
| 80 | 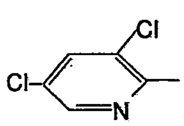 | 4–Cl | $–(CH_2)_3–$ | $–N(CH_3)_2$ | |

| Nr. | Z | $R_n$ | Q | A | phys. Daten |
|---|---|---|---|---|---|
| 81 | 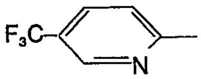 | H | $-(CH_2)_2-$ | $-N(C_2H_5)_2$ | |
| 82 | 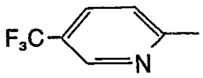 | $4-NO_2$ | $-(CH_2)_2-$ | $-N(C_2H_5)_2$ | |
| 83 | 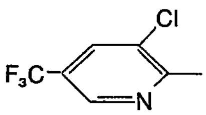 | H | $-(CH_2)_2-$ | $-N(C_2H_5)_2$ | |
| 84 | 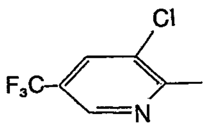 | H | $-(CH_2)_3-$ | $-N(C_2H_5)_2$ | |
| 85 | 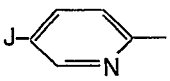 | H | $-(CH_2)_2-$ | $-N(C_2H_5)_2$ | |
| 86 | 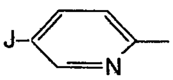 | H | $-(CH_2)_3-$ | $-N(C_2H_5)_2$ | |
| 87 | 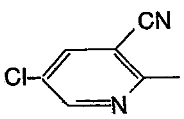 | H | $-(CH_2)_2-$ | $-N(C_2H_5)_2$ | |
| 88 | 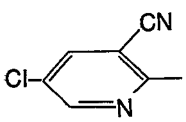 | $4-CH_3$ | $-(CH_2)_2-$ | $-N(C_2H_5)_2$ | |
| 89 | 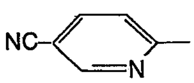 | H | $-(CH_2)_2-$ | $-N(C_2H_5)_2$ | |
| 90 | 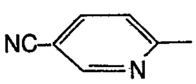 | H | $-(CH_2)_3-$ | $-N(CH_3)_2$ | |
| 91 | 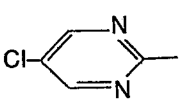 | H | $-(CH_2)_3-$ | $-N(CH_3)_2$ | |
| 92 | 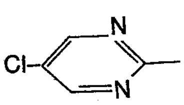 | H | $-(CH_2)_2-$ | $-N(C_2H_5)_2$ | |
| 93 | 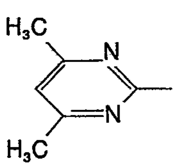 | H | $-(CH_2)_2-$ | $-N(C_2H_5)_2$ | |

| Nr. | Z | $R_n$ | Q | A | phys. Daten |
|---|---|---|---|---|---|
| 94 | 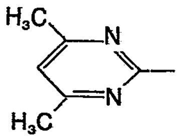 | H | $-(CH_2)_3-$ | $-N(C_2H_5)_2$ | |
| 95 | 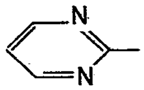 | H | $-(CH_2)_2-$ | $-N(C_2H_5)_2$ | |
| 96 | 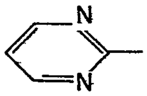 | H | $-(CH_2)_2-$ | $-N(C_2H_5)_2$ | |
| 97 | 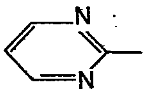 | 4–Cl | $-(CH_2)_2-$ | $-N(C_2H_5)_2$ | |
| 98 | 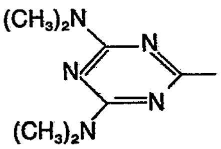 | H | $-(CH_2)_2-$ | $-N(C_2H_5)_2$ | |
| 99 | 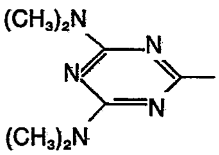 | H | $-(CH_2)_3-$ | $-N(C_2H_5)_2$ | |
| 100 | 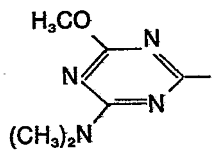 | H | $-(CH_2)_3-$ | $-N(C_2H_5)_2$ | |
| 101 | 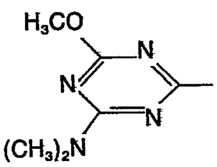 | H | $-(CH_2)_2-$ | $-N(C_2H_5)_2$ | |
| 102 | 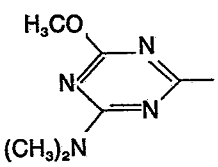 | $4-NO_2$ | $-(CH_2)_2-$ | $-N(C_2H_5)_2$ | |
| 103 | 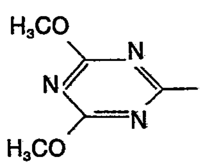 | H | $-(CH_2)_2-$ | $-N(C_2H_5)_2$ | |
| 104 | 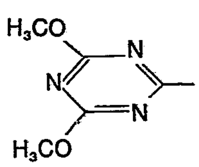 | H | $-(CH_2)_3-$ | $-N(C_2H_5)_2$ | |

| Nr. | Z | $R_n$ | Q | A | phys. Daten |
|---|---|---|---|---|---|
| 105 | 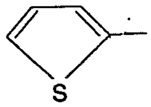 | H | $-(CH_2)_3-$ | $-N(C_2H_5)_2$ | |
| 106 | 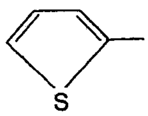 | H | $-(CH_2)_2-$ | $-N(C_2H_5)_2$ | |
| 107 | 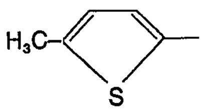 | H | $-(CH_2)_2-$ | $-N(C_2H_5)_2$ | |
| 108 | 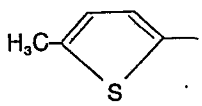 | 4–Cl | $-(CH_2)_2-$ | $-N(C_2H_5)_2$ | |
| 109 | 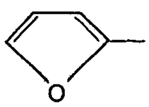 | H | $-(CH_2)_2-$ | $-N(C_2H_5)_2$ | |
| 110 | 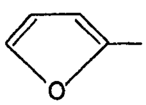 | H | $-(CH_2)_2-$ | $-N(C_2H_5)_2$ | |
| 111 | 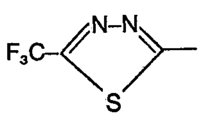 | H | $-(CH_2)_3-$ | $-N(C_2H_5)_2$ | |
| 112 | 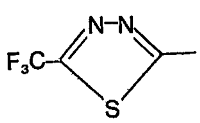 | H | $-(CH_2)_2-$ | $-N(C_2H_5)_2$ | |
| 113 | 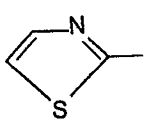 | H | $-(CH_2)_3-$ | $-N(C_2H_5)_2$ | |
| 114 | 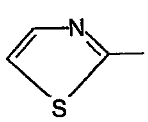 | H | $-(CH_2)_3-$ | $-N(C_2H_5)_2$ | |
| 115 | 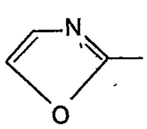 | H | $-(CH_2)_3-$ | $-N(C_2H_5)_2$ | |
| 116 | 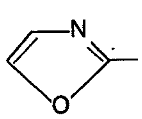 | H | $-(CH_2)_2-$ | $-N(C_2H_5)_2$ | |
| 117 | α-Naphthyl | H | $-(CH_2)_2-$ | $-N(C_2H_5)_2$ | |
| 118 | α-Naphthyl | H | $-(CH_2)_3-$ | $-N(C_2H_5)_2$ | |

| Nr. | Z | $R_n$ | Q | A | phys. Daten |
|---|---|---|---|---|---|
| 119 | α-Naphthyl | 4–Cl | $-(CH_2)_2-$ | $-N(C_2H_5)_2$ | |
| 120 | α-Naphthyl | $4-NO_2$ | $-(CH_2)_2-$ | $-N(C_2H_5)_2$ | |
| 121 | α-Naphthyl | $4-OCH_3$ | $-(CH_3)_2-$ | $-N(C_2H_5)_2$ | |
| 122 | β-Naphthyl | H | $-(CH_2)_2-$ | $-N(C_2H_5)_2$ | |
| 123 | β-Naphthyl | H | $-(CH_2)_3-$ | $-N(C_2H_5)_2$ | |
| 124 | β-Naphthyl | 4–Cl | $-(CH_2)_2-$ | $-N(C_2H_5)_2$ | |
| 125 | β-Naphthyl | $4-NO_2$ | $-(CH_2)_2-$ | $-N(C_2H_5)_2$ | |
| 126 | β-Naphthyl | 4–CN | $-(CH_2)_2-$ | $-N(C_2H_5)_2$ | |
| 127 | 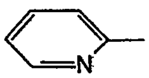 | H | $-(CH_2)_2-$ | $-N(C_2H_5)_2$ | |
| 128 | $C_6H_5-$ | H | $-(CH_2)_2-$ | $-N(C_2H_5)-CH_2-COOC_2H_5$ | |
| 129 | $C_6H_5-$ | H | $-(CH_2)_2-$ | $-N(C_2H_5)-CH_2-COOH$ | |
| 130 | $C_6H_5-$ | H | $-(CH_2)_2-$ | $-N(CH_2-CH_2OH)_2$ | |
| 131 | $C_6H_5-$ | H | $-(CH_2)_2-$ | $-N(C_2H_5)-CH_2-CH_2-O-CH_3$ | |
| 132 | $C_6H_5-$ | H | $-CH-CH_2-$<br>$\vert$<br>$CH_3$ | $-N(CH_3)_2$ | |
| 133 | $C_6H_5-$ | H | $-CH-CH_2-$<br>$\vert$<br>$CH_3$ | $-N(C_2H_5)_2$ | |
| 134 | $C_6H_5-$ | H | $-CH_2-CH-$<br>$\vert$<br>$CH_3$ | $-N(CH_3)_2$ | |
| 135 | $C_6H_5-$ | H | $-CH_2-CH-$<br>$\vert$<br>$CH_3$ | $-N-C_2H_5$<br>$\vert$<br>$CH_3$ | |
| 136 | $C_6H_5-$ | H | $-CH-CH-$<br>$\vert\quad\vert$<br>$CH_3\ CH_3$ | $-N-C_2H_5$<br>$\vert$<br>$CH_3$ | |
| 137 | $C_6H_5-$ | 4 – CN | $-CH_2-CH-$<br>$\vert$<br>$C_2H_5$ | $-N(CH_3)_2$ | |
| 138 | $C_6H_5-$ | 4–Cl | $-(CH_2)_2-CH-$<br>$\vert$<br>$CH_3$ | $-N-C_3H_7(n)$<br>$\vert$<br>$CH_3$ | |
| 139 | $4-F-C_6H_4-$ | H | $-CH-CH_2-$<br>$\vert$<br>$CH_3$ | $-N(CH_3)_2$ | |
| 140 | $4-F-C_6H_4-$ | H | $-CH-CH_2)_2-$<br>$\vert$<br>$CH_3$ | $-N(C_2H_5)_2$ | |

| Nr. | Z | $R_n$ | Q | A | phys. Daten |
|---|---|---|---|---|---|
| 141 | $4-F-C_6H_4-$ | H | $-CH_2-CH-CH_2-$<br>\|<br>$CH_3$ | $-N(C_2H_5)_2$ | |
| 142 | $4-F-C_6H_4-$ | 4–CN | $-CH-CH_2-$<br>\|<br>$C_2H_5$ | $-N(CH_3)_2$ | |
| 143 | $4-OCH_3-C_6H_4-$ | H | $-CH-CH-$<br>\| \|<br>$CH_3$ $CH_3$ | $-N(CH_3)_2$ | |
| 144 | $4-OCH_3-C_6H_4-$ | H | $-CH_2-CH-$<br>\|<br>$CH_3$ | $-N(C_2H_5)_2$ | |
| 145 | $4-OCH_3-C_6H_4-$ | 4–Cl | $-CH-CH_2-$<br>\|<br>$CH_3$ | $-NHC_3H_7(i)$ | |
| 146 | $4-OCH_3-C_6H_4-$ | 2–Cl | $-(CH_2)_3-CH-$<br>\|<br>$CH_3$ | $-N(C_2H_5)_2$ | |
| 147 | 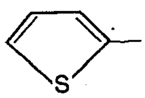 | H | $-CH-CH_2-$<br>\|<br>$CH_3$ | $-N(CH_3)_2$ | |
| 148 | 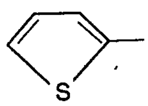 | H | $-CH_2-CH-$<br>\|<br>$CH_3$ | $-N(CH_3)_2$ | |
| 149 | $H_3C-$ 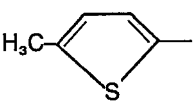 | H | $-CH-CH_2-$<br>\|<br>$CH_3$ | $-N(C_2H_5)_2$ | |
| 150 | $H_3C-$ 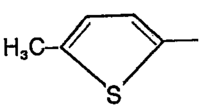 | 4–CN | $-CH_2-CH-$<br>\|<br>$CH_3$ | $-N(C_2H_5)_2$ | |
| 151 | α-Naphthyl | H | $-CH-CH_2-$<br>\|<br>$CH_3$ | $-N(C_2H_5)_2$ | |
| 152 | β-Naphthyl | H | $-(CH_2)_2-$ | $-N(CH_3)_2$ | |
| 153 | $C_6H_5-$ | H | $-(CH_2)_4-$ | $-N(C_2H_5)_2$ | |
| 154 | $4-F-C_6H_4-$ | H | $-(CH_2)_4-$ | $-N(C_2H_5)_2$ | |
| 155 | $4-OCH_3-C_6H_4-$ | H | $-(CH_2)_4-$ | $-N(C_2H_5)_2$ | |
| 156 | $4-Cl-C_6H_4-$ | H | $-(CH_2)_4-$ | $-N(C_2H_5)_2$ | |
| 157 | $2-F-4-F-C_6H_3-$ | H | $-(CH_2)_4-$ | $-N(C_2H_5)_2$ | |

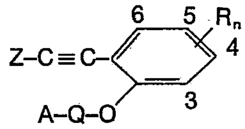

| Nr. | Z | $R_n$ | Q | A | phys. Daten |
|---|---|---|---|---|---|
| 201 | $C_6H_5-$ | H | $-(CH_2)_2-$ | $-N(C_2H_5)_2$ | $n_D^{25}$: 1,5988 |
| 202 | $C_6H_5-$ | H | $-(CH_2)_3-$ | $-N(C_2H_5)_2$ | |
| 203 | $C_6H_5-$ | $5-NO_2$ | $-(CH_2)_2-$ | $-N(C_2H_5)_2$ | |
| 204 | $C_6H_5-$ | $3-NO_2$ | $-(CH_2)_2-$ | $-N(C_2H_5)_2$ | |
| 205 | $C_6H_5-$ | 5–Cl | $-(CH_2)_2-$ | $-N(C_2H_5)_2$ | |
| 206 | $C_6H_5-$ | 3–Cl | $-(CH_2)_2-$ | $-NHC_2H_5$ | |
| 207 | $C_6H_5-$ | 5–CN | $-(CH_2)_2-$ | $-N(C_2H_5)_2$ | |
| 208 | $C_6H_5-$ | 3–CN | $-(CH_2)_2-$ | $-N(C_2H_5)_2$ | |
| 209 | $C_6H_5-$ | $3-NO_2$ | $-(CH_2)_3-$ | $-N(C_2H_5)_2$ | |
| 210 | $4-CF_3-2-Cl-C_6H_3-$ | H | $-(CH_2)_2-$ | $-N(nC_3H_7)2$ | |
| 211 | $4-CF_3-2-Cl-C_6H_3-$ | 5–CN | $-(CH_2)_2-$ | $-N(C_2H_5)_2$ | |
| 212 | $3-CH_3-5-CH_3-C_6H_3-$ | H | $-(CH_2)_2-$ | $-N(C_2H_5)_2$ | |
| 213 | $4-iC_3H_7-C_6H_4-$ | H | $-(CH_2)_2-$ | $-N(C_2H_5)_2$ | |
| 214 | $4-iC_3H_7-C_6H_4-$ | H | $-(CH_2)_2-$ | $-N(nC_4H_9)_2$ | |
| 215 | $4-CN-C_6H_4-$ | H | $-(CH_2)_2-$ | $-N(C_2H_5)_2$ | |
| 216 | $4-CN-C_6H_4-$ | 3–Cl | $-(CH_2)_2-$ | $-N(C_2H_5)_2$ | |
| 217 | $4-NO_2-C_6H_4$ | H | $-(CH_2)_2-$ | $-N(C_2H_5)_2$ | |
| 218 | $2-C_2H_5-6-CH_3-C_6H_3-$ | H | $-(CH_2)_2-$ | $-N(C_2H_5)_2$ | |
| 219 | $2-C_2H_5-6-CH_3-C_6H_3-$ | $3-NO_2$ | $-(CH_2)_2-$ | $-N(OCH_3)CH_3$ | |
| 220 | $4-OCH_3-C_6H_4-$ | H | $-(CH_2)_2-$ | $-N(C_2H_5)_2$ | |
| 221 | $4-SC_2H_5-C_6H_4$ | H | $-(CH_2)_3-$ | $-N(nC_3H_7)_2$ | |
| 222 | $4-SC_2H_5-C_6H_4-$ | $5-NO_2$ | $-(CH_2)_2-$ | $-N(C_2H_5)_2$ | |
| 223 | $3-OCH_3-C_6H_4-$ | H | $-(CH_2)_2-$ | $-N(C_2H_5)_2$ | |
| 224 | 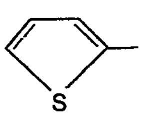 | H | $-(CH_2)_2-$ | $-N(C_2H_5)_2$ | |
| 225 | 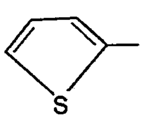 | H | $-(CH_2)_2-$ | $-N(C_2H_5)_2$ | |

| Nr. | Z | $R_n$ | Q | A | phys. Daten |
|---|---|---|---|---|---|
| 226 | 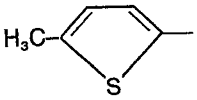 | H | $-(CH_2)_3-$ | $-N(C_2H_5)_2$ | |
| 227 | 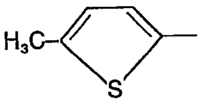 | H | $-(CH_2)_2-$ | $-N(C_2H_5)_2$ | |

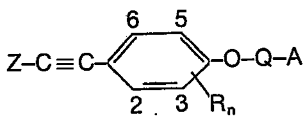

| Nr. | Z | $R_n$ | Q | A | phys. Daten |
|---|---|---|---|---|---|
| 401 | $C_6H_5-$ | H | $-(CH_2)_2-$ | $-N(C_2H_5)_2 \cdot HCl$ | |
| 402 | $C_6H_5-$ | H | $-(CH_2)_2-$ | $-N(CH_3)-C_2H_5$ | $n_D^{25}$: 1,6203 |
| 403 | $C_6H_5-$ | H | $-(CH_2)_6-$ | $-N(C_2H_5)_2$ | |
| 404 | $C_6H_5-$ | H | $-(CH_2)_2-$ | $-NHC_2H_5$ | viskos |
| 405 | $C_6H_5-$ | H | $-(CH_2)_2-$ | $-N(CH_3)_2$ | Smp. 34–36° |
| 406 | $C_6H_5-$ | H | $-(CH_2)_2-$ | 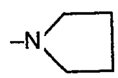 | viskos |
| 407 | $C_6H_5-$ | 2–Cl,5–Cl | $-(CH_2)_2-$ | $-N(C_2H_5)_2$ | $n_D^{25}$: 1,6173 |
| 408 | $C_6H_5-$ | H | $-(CH_2)_3-$ | $-N(C_2H_5)_2$ | $n_D^{25}$: 1,5934 |
| 409 | $C_6H_5-$ | H | $-(CH_2)_2-$ | 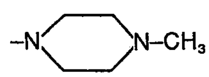 | Smp. 65–70° |
| 410 | $C_6H_5-$ | 3–Cl,5–Cl | $-(CH_2)_2-$ | $-N(C_2H_5)_2$ | |
| 411 | $C_6H_5-$ | $2-CH_3,5-CH_3$ | $-(CH_2)_2-$ | $-N(C_2H_5)_2$ | |
| 412 | $C_6H_5-$ | $5-NO_2$ | $-(CH_2)_2-$ | $-N(C_2H_5)_2$ | |
| 413 | $C_6H_5-$ | 2–Cl,5–Cl | $-(CH_2)_3-$ | $-N(CH_3)_2$ | |
| 414 | $C_6H_5-$ | 2–Cl,6–Cl | $-(CH_2)_3-$ | $-N(C_2H_5)_2$ | |
| 415 | $C_6H_5-$ | $5-NO_2$ | $-(CH_2)_3-$ | $-N(C_2H_5)_2$ | |
| 416 | $4-F-C_6H_4-$ | H | $-(CH_2)_2-$ | $-N(C_2H_5)_2$ | Sdp. 156–162°/0,08 mb |
| 417 | $4-Cl-C_6H_4-$ | H | $-(CH_2)_3-$ | $-N(C_2H_5)_2$ | Smp. 69–70° |
| 418 | $4-CF_3-C_6H_4-$ | H | $-(CH_2)_2-$ | $-N(C_2H_5)_2$ | |

| Nr. | Z | $R_n$ | Q | A | phys. Daten |
|---|---|---|---|---|---|
| 419 | $4-SCH_3-C_6H_4-$ | H | $-(CH_2)_2-$ | $-N(C_2H_5)_2$ | |
| 420 | $4-CF_3-2-Cl-C_6H_3-$ | $5-NO_2$ | $-(CH_2)_2-$ | $-N(C_2H_5)_2$ | |
| 421 | $4-CF_3-2-Cl-C_6H_3-$ | $5-NO_2$ | $-(CH_2)_3-$ | $-N(C_2H_5)_2$ | |
| 422 | $3-CH_3-5-CH_3-C_6H_3-$ | H | $-(CH_2)_2-$ | $-N(C_2H_5)_2$ | $n_D^{20}$: 1,5880 |
| 423 | $4-iC_3H_7-C_6H_4-$ | H | $-(CH_2)_2-$ | $-N(C_2H_5)_2$ | Smp. 50° |
| 424 | $4-iC_3H_7-C_6H_4-$ | 2–Cl, 5–Cl | $-(CH_2)_3-$ | $-N(C_2H_5)_2$ | |
| 425 | $4-CN-C_6H_4-$ | H | $-(CH_2)_2-$ | $-N(C_2H_5)_2$ | |
| 426 | $4-CN-C_6H_4$ | 3-Cl,5-Cl | $-(CH_2)_2-$ | $-N(C_2H_5)_2$ | |
| 427 | $4-NO_2-C_6H_4-$ | H | $-(CH_2)_2-$ | $-N(C_2H_5)_2$ | Smp. 58° |
| 428 | $3-NO_2-C_6H_4-$ | H | $-(CH_2)_2-$ | $-N(C_2H_5)_2$ | $n_D^{30}$: 1,5980 |
| 429 | $2-NO_2-4-Cl-C_6H_3-$ | H | $-(CH_2)_2-$ | $-N(C_2H_5)_2$ | |
| 430 | $2-NO_2-4-Cl-C_6H_3-$ | $2-CH_3,5-CH_3$ | $-(CH_2)_2-$ | $-N(C_2H_5)_2$ | |
| 431 | $2-C_2H_5-6-CH_3-C_6H_3-$ | H | $-(CH_2)_2-$ | $-N(C_2H_5)_2$ | $n_D^{30}$: 1,5820 |
| 432 | $C_6F_5-$ | H | $-(CH_2)_2-$ | $-N(C_2H_5)_2$ | Smp. 65° |
| 433 | $4-OCH_3-C_6H_4-$ | H | $-(CH_2)_2-$ | $-N(C_2H_5)_2$ | Smp. 59–60° |
| 434 | $4-OCH_3-C_6H_4$ | H | $-(CH_2)_2-$ | $-N(CH_3)-C_2H_5$ | Smp. 40–45° |
| 435 | $4-SC_2H_5-C_6H_4-$ | H | $-(CH_2)_2-$ | $-N(C_2H_5)_2$ | |
| 436 | $3-CH_3-5-OH-C_6H_3-$ | H | $-(CH_2)_2-$ | $-N(C_2H_5)_2$ | Öl |
| 437 | $3-CF_3-C_6H_4-$ | H | $-(CH_2)_2-$ | $-N(nC_3H_7)_2$ | |
| 438 | $3-CF_3-C_6H_4-$ | H | $-(CH_2)_4-$ | $-N(C_2H_5)_2$ | |
| 439 | $3-CF_3-5-CF_3-C_6H_3-$ | H | $-(CH_2)_2-$ | $-N(C_2H_5)_2$ | |
| 440 | $3-CF_3-5-CF_3-C_6H_3-$ | H | $-(CH_2)_2-$ | $-N(OCH_3)CH_3$ | |
| 441 | $3-H_2N-CO-C_6H_4-$ | H | $-(CH_2)_2-$ | $-N(C_2H_5)_2$ | Smp. 133° |
| 442 | $3-CN-C_6H_4-$ | H | $-(CH_2)_2-$ | $-N(C_2H_5)_2$ | |
| 443 | $3-COOCH_3-C_6H_4-$ | H | $-(CH_2)_2-$ | $-N(C_2H_5)_2$ | |
| 444 | $3-COOCH_3-C_6H_4$ | 3-Cl,5-Cl | $-(CH_2)_2-$ | $-N(C_2H_5)_2$ | |
| 445 | $2-OCH_3-C_6H_4-$ | H | $-(CH_2)_2-$ | $-N(CH_3)_2$ | Öl |
| 446 | $3-OCH_3-C_6H_4-$ | $2-CH_3,5-CH_3$ | $-(CH_2)_3-$ | $-N(CH_3)_2$ | |
| 447 | $3-CON(CH_3)_2-C_6H_4-$ | H | $-(CH_2)_2-$ | $-N(C_2H_5)_2$ | |
| 448 | $3-COSC_2H_5-C_6H_4-$ | H | $-(CH_2)_2-$ | $-N(C_2H_5)_2$ | |

| Nr. | Z | $R_n$ | Q | A | phys. Daten |
|---|---|---|---|---|---|
| 449 | 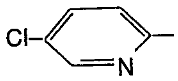 | H | $-(CH_2)_2-$ | $-N(C_2H_5)_2$ | |
| 450 | 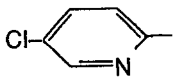 | H | $-(CH_2)_3-$ | $-N(C_2H_5)_2$ | |
| 451 | 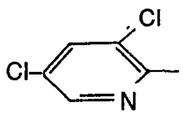 | 5–$NO_2$ | $-(CH_2)_3-$ | $-N(C_2H_5)_2$ | |
| 452 | 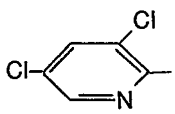 | H | $-(CH_2)_2-$ | $-N(C_2H_5)_2$ | Smp. 58° |
| 453 | 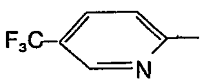 | H | $-(CH_2)_2-$ | $-N(C_2H_5)_2$ | |
| 454 | 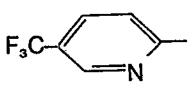 | H | $-(CH_2)_2-$ | 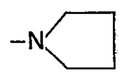 | |
| 455 | 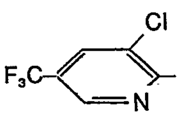 | H | $-(CH_2)_2-$ | $-N(C_2H_5)_2$ | |
| 456 | 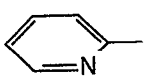 | H | $-(CH_2)_2-$ | $-N(C_2H_5)_2$ | $n_D^{30}$: 1,6040 |
| 457 | 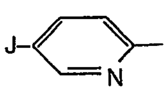 | H | $-(CH_2)_2-$ | $-N(C_2H_5)_2$ | |
| 458 | 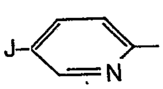 | 2–Cl,6–Cl | $-(CH_2)_2-$ | $-N(C_2H_5)_2$ | |
| 459 | 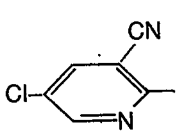 | H | $-(CH_2)_2-$ | $-N(C_2H_5)_2$ | |
| 460 | 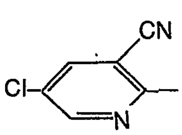 | H | $-(CH_2)_3-$ | $-N(C_2H_5)_2$ | |
| 461 | 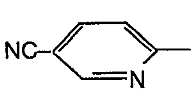 | 3–Cl,5–Cl | $-(CH_2)_2-$ | $-N(C_2H_5)_2$ | |

| Nr. | Z | $R_n$ | Q | A | phys. Daten |
|---|---|---|---|---|---|
| 462 | 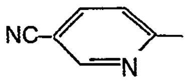 | H | $-(CH_2)_2-$ | $-N(C_2H_5)_2$ | |
| 463 | 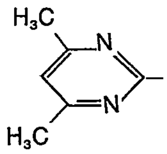 | H | $-(CH_2)_2-$ | $-N(C_2H_5)_2$ | |
| 464 | 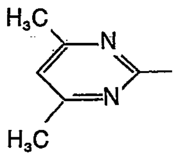 | H | $-(CH_2)_3-$ | $-N(OCH_3)CH_3$ | |
| 465 | 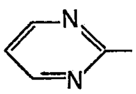 | H | $-(CH_2)_2-$ | $-N(C_2H_5)_2$ | |
| 466 | 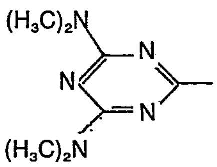 | H | $-(CH_2)_2-$ | $-N(C_2H_5)_2$ | |
| 467 | 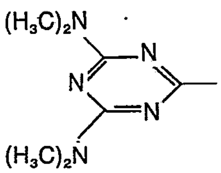 | H | $-(CH_2)_2-$ | $-N(CH_3)_2$ | |
| 468 | 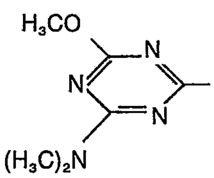 | H | $-(CH_2)_2-$ | $-N(C_2H_5)_2$ | |
| 469 | 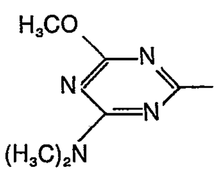 | H | $-(CH_2)_3-$ | $-N(C_2H_5)_2$ | |
| 470 | 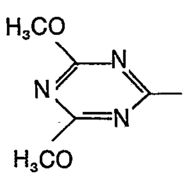 | H | $-(CH_2)_3-$ | $-N(C_2H_5)_2$ | |

| Nr. | Z | $R_n$ | Q | A | phys. Daten |
|---|---|---|---|---|---|
| 471 | 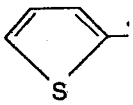 | H | $-(CH_2)_2-$ | $-N(C_2H_5)_2$ | $n_D^{30}$: 1,6170 |
| 472 | 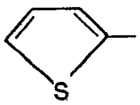 | 2–Cl,5–Cl | $-(CH_2)_2-$ | $-N(C_2H_5)_2$ | |
| 473 | 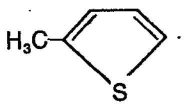 | H | $-(CH_2)_2-$ | $-N(C_2H_5)_2$ | |
| 474 | 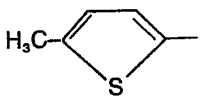 | H | $-(CH_2)_3-$ | $-N(C_2H_5)_2$ | |
| 475 | 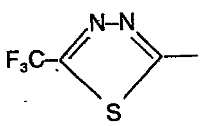 | H | $-(CH_2)_2-$ | $-N(C_2H_5)_2$ | |
| 476 | 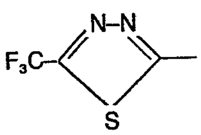 | H | $-(CH_2)_2-$ | $-N(OCH_3)CH_3$ | |
| 477 | 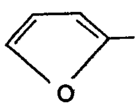 | H | $-(CH_2)_2-$ | $-N(C_2H_5)_2$ | |
| 478 | 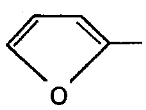 | 2–Cl,6–Cl | $-(CH_2)_3-$ | | |
| 479 | 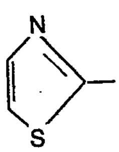 | H | $-(CH_2)_2-$ | $-N(C_2H_5)_2$ | |
| 480 | 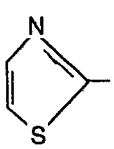 | 2–$CH_3$,5–$CH_3$ | $-(CH_2)_2-$ | $-N(C_2H_5)_2$ | |
| 481 | 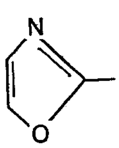 | H | $-(CH_2)_2-$ | $-N(C_2H_5)_2$ | |
| 482 | α-Naphthyl | H | $-(CH_2)_2-$ | $-N(C_2H_5)_2$ | $n_D^{30}$: 1,6310 |
| 483 | α-Naphthyl | 2–$CH_3$,5–$CH_3$ | $-(CH_2)_2-$ | $-N(C_2H_5)_2$ | |
| 484 | α-Naphthyl | H | $-(CH_2)_3-$ | $-N(C_2H_5)_2$ | |

| Nr. | Z | $R_n$ | Q | A | phys. Daten |
|---|---|---|---|---|---|
| 485 | β-Naphthyl | H | $-(CH_2)_3-$ | $-N(C_2H_5)_2$ | |
| 486 | β-Naphthyl | $5-NO_2$ | $-(CH_2)_3-$ | $-N(C_2H_5)_2$ | |
| 487 | 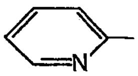 | H | $-(CH_2)_2-$ | $-N(C_2H_5)_2$ | |
| 488 | $C_6H_5-$ | H | $-(CH_2)_2-$ | $-N(C_2H_5)-CH_2-COOC_2H_5$ | Wachs |
| 489 | $C_6H_5-$ | H | $-(CH_2)_2-$ | $-N(C_2H_5)-CH_2-COOH$ | |
| 490 | $C_6H_5-$ | H | $-(CH_2)_2-$ | $-N(CH_2-COOCH_3)_2$ | |
| 491 | $C_6H_5-$ | H | $-(CH_2)_2-$ | $-N(C_2H_5)-CH_2-CH_2OH$ | |
| 492 | $C_6H_5-$ | H | $-CH-CH_2-$<br>\|<br>$CH_3$ | $-N(CH_3)_2$ | $n_D^{25}$: 1,6091 |
| 493 | $C_6H_5-$ | H | $-CH_2-CH-$<br>\|<br>$CH_3$ | $-N(CH_3)_2$ | $n_D^{25}$: 1,6122 |
| 494 | $C_6H_5-$ | H | $-CH-CH_2-$<br>\|<br>$C_2H_5$ | $-N(CH_3)_2$ | |
| 495 | $C_6H_5-$ | H | $-CH-CH_2-$<br>\|<br>$CH_3$ | $-N(C_2H_5)_2$ | |
| 496 | $C_6H_5-$ | H | $-CH-CH_2-$<br>\|<br>$CH_3$ | $-N-C_2H_5$<br>\|<br>$CH_3$ | |
| 497 | $C_6H_5-$ | H | $-CH-(CH_2)_4-$<br>\|<br>$CH_3$ | $-N(CH_3)_2$ | |
| 498 | $C_6H_5-$ | H | $-CH_2-CH-$<br>\|<br>$CH_3$ | $-N(C_2H_5)_2$ | |
| 499 | $C_6H_5-$ | H | $-CH_2-CH-$<br>\|<br>$C_2H_5$ | $-N(CH_3)_2$ | |
| 500 | $C_6H_5-$ | H | $-CH-CH_2-CH-$<br>\| \|<br>$CH_3$ $CH_3$ | $-N(CH_2-CH=CH_2)_2$ | |
| 501 | $C_6H_5-$ | H | $-CH-CH-$<br>\| \|<br>$CH_3$ $CH_3$ | $-N-C_2H_5$<br>\|<br>$CH_3$ | |
| 502 | $C_6H_5-$ | $2-Cl$ | $-CH-CH_2-$<br>\|<br>$CH_3$ | $-N-C_4H_9(n)$<br>\|<br>$CH_3$ | |

| Nr. | Z | $R_n$ | Q | A | phys. Daten |
|---|---|---|---|---|---|
| 503 | $C_6H_5-$ | $5-CH_3$ | 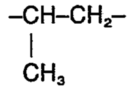 | $-N(CH_3)_2$ | |
| 504 | $C_6H_5-$ | $5-CH_3$ | 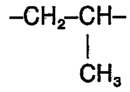 | $-N(C_2H_5)_2$ | |
| 505 | $4-F-C_6H_4-$ | H | 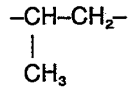 | $-N(CH_3)_2$ | |
| 506 | $4-F-C_6H_4-$ | H | 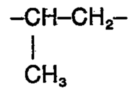 | $-N(C_2H_5)_2$ | |
| 507 | $4-F-C_6H_4-$ | H | 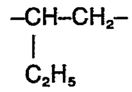 | $-N(CH_3)_2$ | |
| 508 | $4-F-C_6H_4-$ | H | 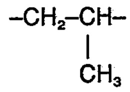 | $-N(CH_3)_2$ | |
| 509 | $4-F-C_6H_4-$ | H | 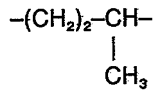 | 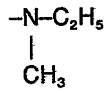 | |
| 510 | $4-F-C_6H_4-$ | H | 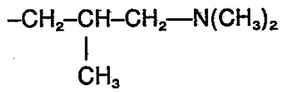 | | |
| 511 | $4-OCH_3-C_6H_4-$ | $5-CH_3$ | 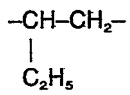 | $-N(CH_3)_2$ | |
| 512 | $4-OCH_3-C_6H_4-$ | H | 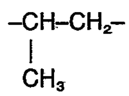 | $-N(CH_3)_2$ | |
| 513 | $4-OCH_3-C_6H_4-$ | H | 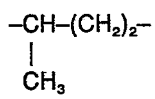 | 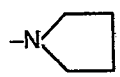 | |
| 514 | $4-OCH_3-C_6H_4-$ | 2-Cl | 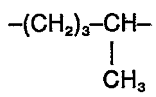 | $-N(CH_3)_2$ | |
| 515 | $4-OCH_3-C_6H_4-$ | H | 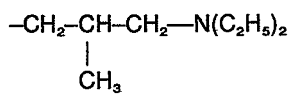 | | |
| 516 | $4-OCH_3-C_6H_4-$ | H | 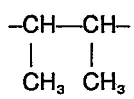 | $-N(CH_3)_2$ | |
| 517 | $4-OCH_3-C_6H_4-$ | H | 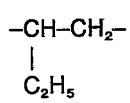 | $-N(C_2H_5)_2$ | |

| Nr. | Z | $R_n$ | Q | A | phys. Daten |
|---|---|---|---|---|---|
| 518 | 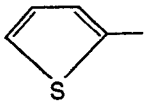 | H | $-CH-CH_2-$<br>$\vert$<br>$CH_3$ | $-N(CH_3)_2$ | |
| 519 | 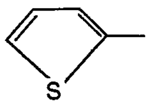 | H | $-CH_2-CH-$<br>$\vert$<br>$CH_3$ | $-N(CH_3)_2$ | |
| 520 | 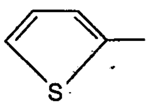 | H | $-CH_2CH-$<br>$\vert$<br>$C_2H_5$ | $-N-C_2H_5$<br>$\vert$<br>$CH_3$ | |
| 521 | 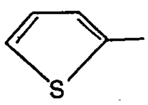 | H | $-CH-(CH_2)_2-$<br>$\vert$<br>$CH_3$ | $-N(C_3H_7-n)_2$ | |
| 522 | 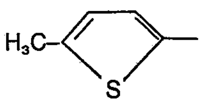 | $5-CH_3$ | $-CH_2-CH-CH_2-$<br>$\vert$<br>$CH_3$ | $-NHC_4H_9(n)$ | |
| 523 | 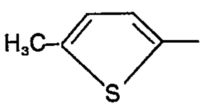 | H | $-CH-CH_2-$<br>$\vert$<br>$CH_2$ | $-N(C_2H_5)_2$ | |
| 524 | 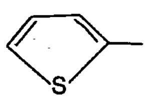 | 2–Cl | $-CH-CH_2-$<br>$\vert$<br>$C_2H_5$ | $-N(CH_3)_2$ | |
| 525 | α-Naphthyl | H | $-CH-CH_2-$<br>$\vert$<br>$C_2H_5$ | 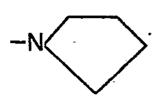 | |
| 526 | α-Naphthyl | H | $-CH_2-CH-$<br>$\vert$<br>$CH_3$ | $-N(CH_3)_2$ | |
| 527 | β-Naphthyl | $5-CH_3$ | $-CH_2-CH-$<br>$\vert$<br>$CH_3$ | $-N(CH_3)_2$ | |
| 528 | β-Naphthyl | H | $-CH-CH-$<br>$\vert \quad \vert$<br>$CH_3 \; CH_3$ | $-N(C_2H_5)_2$ | |
| 529 | $4-C_2H_5-O-CO-NH-C_6H_4-$ | H | $-(CH_2)_2-$ | $-N(C_2H_5)_2$ | Smp. 92° |
| 530 | $4-CF_3-CO-NH-C_6H_4$ | H | $-(CH_2)_2-$ | $-N(C_2H_5)_2$ | Smp. 140° |

| Nr. | Z | $R_n$ | Q | A | phys. Daten |
|---|---|---|---|---|---|
| 531 | $4-C_3H_7-i-CO-NH-C_6H_4-$ | H | $-(CH_2)_2-$ | $-N(C_2H_5)_2$ | Smp. 147° |
| 532 | $4-CH_3-O-CO-NH-C_6H_4-$ | H | $-(CH_2)_2-$ | $-N(C_2H_5)_2$ | Smp. 84° |
| 533 | $3-(CH_3)_2N-CH=N-C_6H_4-$ | H | $-(CH_2)_2-$ | $-N(C_2H_5)_2$ | Smp. 37–38° |
| 534 | $3-N_3-C_6H_4-$ | H | $-(CH_2)_2-$ | $-N(C_2H_5)_2$ | |
| 535 | $4-N_3-C_6H_4-$ | H | $-(CH_2)_2-$ | $-N(C_2H_5)_2$ | |
| 536 | $4-(C_2H_5)_2N-SO_2-C_6H_4-$ | H | $-(CH_2)_2-$ | $-N(C_2H_5)_2$ | |
| 537 | $4-(CH_3)_2N-C_6H_4-$ | H | $-(CH_2)_2-$ | $-N(C_2H_5)_2$ | |
| 538 | $4-CH_3-O-CH_2-C_6H_4-$ | H | $-(CH_2)_2-$ | $-N(C_2H_5)_2$ | |
| 539 | $4-CN-CH_2-C_6H_4-$ | H | $-(CH_2)_2-$ | $-N(C_2H_5)_2$ | |
| 540 | $4-HOOC-(CH_2)_2-C_6H_4-$ | H | $-(CH_2)_2-$ | $-N(C_2H_5)_2$ | |
| 541 | $4-C_2H_5-NH-C_6H_4-$ | H | $-(CH_2)_2-$ | $-N(C_2H_5)_2$ | |
| 542 | $4-Cl-CH_2-C_6H_4-$ | H | $-(CH_2)_2-$ | $-N(C_2H_5)_2$ | |
| 543 | $4-CH_3-O-CO-C_6H_4-$ | H | $-(CH_2)_2-$ | $-N(C_2H_5)_2$ | |
| 544 | $4-SCH_3-C_6H_4-$ | H | $-(CH_2)_2-$ | $-N(C_2H_5)_2$ | |
| 545 | $4-CH_2F-O-C_6H_4-$ | H | $-(CH_2)_2-$ | $-N(C_2H_5)_2$ | |
| 546 | $3-OCH_3-C_6H_4-$ | H | $-(CH_2)_2-$ | $-N(C_2H_5)_2$ | |
| 547 | $3-(CH_3)_2N-C_6H_4-$ | H | $-(CH_2)_2-$ | $-N(C_2H_5)_2$ | |
| 548 | $3-C_2H_5-O-CO-NH-C_6H_4-$ | H | $-(CH_2)_2-$ | $-N(C_2H_5)_2$ | |
| 549 | $3-C_2H_5-NH-C_6H_4-$ | H | $-(CH_2)_2-$ | $-N(C_2H_5)_2$ | |
| 550 | $C_6H_5-$ | H | $-(CH_2)_4-$ | $-N(C_2H_5)_2$ | |
| 551 | $4-F-C_6H_4-$ | H | $-(CH_2)_4-$ | $-N(C_2H_5)_2$ | |
| 552 | $4-OCH_3-C_6H_4-$ | H | $-(CH_2)_4-$ | $-N(C_2H_5)_2$ | |
| 553 | $4-Cl-C_6H_4-$ | H | $-(CH_2)_4-$ | $-N(C_2H_5)_2$ | |
| 554 | $4-NH_2-C_6H_4-$ | H | $-(CH_2)_2-$ | $-N(C_2H_5)_2$ | Smp. 103° |
| 555 | $3-NH_2-C_6H_4-$ | H | $-(CH_2)_2-$ | $-N(C_2H_5)_2$ | Smp. 51° |
| 556 | $2-NH_2-C_6H_4-$ | H | $-(CH_2)_2-$ | $-N(C_2H_5)_2$ | |
| 557 | $2-NH_2-C_6H_4-$ | H | $-CH(CH_3)-CH_2-$ | $-N(C_2H_5)_2$ | |
| 558 | $4-OH-C_6H_4-$ | H | $-(CH_2)_2-$ | $-N(C_2H_5)_2$ | |
| 559 | $3-OH-C_6H_4-$ | H | $-(CH_2)_2-$ | $-N(C_2H_5)_2$ | |
| 560 | $2-OH-C_6H_4-$ | H | $-(CH_2)_2-$ | $-N(C_2H_5)_2$ | |

| Nr. | Z | $R_n$ | Q | A | phys. Daten |
|---|---|---|---|---|---|
| 561 | $4-OH-C_6H_4-$ | $3-NO_2$ | $-(CH_2)_2-$ | $-N(C_2H_5)_2$ | |
| 562 | $4-C_2H_5-O-C_6H_4-$ | H | $-(CH_2)_2-$ | $-N(C_2H_5)_2$ | |
| 563 | $3-C_2H_5-O-C_6H_4-$ | H | $-(CH_2)_2-$ | $-N(C_2H_5)_2$ | |
| 564 | $4-C_2H_5-O-C_6H_4-$ | $3-NO_2$ | $-(CH_2)_2-$ | $-N(C_2H_5)_2$ | |
| 565 | $4-C_2H_5-O-C_6H_4-$ | H | $-CH-CH_2$<br>$\vert$<br>$CH_3$ | $-N(CH_3)_2$ | |
| 566 | $4-CN-C_6H_4-$ | $3-NO_2$ | $-CH-CH_2-$<br>$\vert$<br>$CH_3$ | $-N(CH_3)_2$ | |
| 567 | $4-CN-C_6H_4-$ | H | $-CH-CH_2-$<br>$\vert$<br>$CH_3$ | $-N(CH_3)_2$ | |
| 568 | $3-CN-C_6H_4-$ | H | $-CH-CH_2-$<br>$\vert$<br>$CH_3$ | $-N(CH_3)_2$ | |
| 569 | $3-CN-C_6H_4-$ | $3-NO_2$ | $-CH-CH_2-$<br>$\vert$<br>$CH_3$ | $-N(CH_3)_2$ | |
| 570 | $4-NaO-CO-C_6H_4-$ | H | $-CH-CH_2-$<br>$\vert$<br>$CH_3$ | $-N(CH_3)_2$ | |
| 571 | $4-NaO-CO-C_6H_4-$ | H | $-(CH_2)_2-$ | $-N(C_2H_5)_2$ | |
| 572 | $3-NaO-CO-C_6H_4-$ | H | $-(CH_2)_2-$ | $-N(C_2H_5)_2$ | |
| 573 | $4-OH-NH-C_6H_4-$ | H | $-(CH_2)_2-$ | $-N(C_2H_5)_2$ | |
| 574 | $3-OH-NH-C_6H_4-$ | H | $-(CH_2)_2-$ | $-N(C_2H_5)_2$ | |
| 575 | $4-NH_2-NH-C_6H_4-$ | H | $-(CH_2)_2-$ | $-N(C_2H_5)_2$ | |
| 576 | $3-NH_2-NH-C_6H_4$ | H | $-(CH_2)_2-$ | $-N(C_2H_5)_2$ | |
| 577 | $4-NH_2-NH-C_6H_4-$ | H | $-CH-CH_2-$<br>$\vert$<br>$CH_3$ | $-N(CH_3)_2$ | |
| 578 | 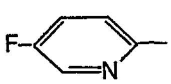 | H | $-(CH_2)_2-$ | $-N(C_2H_5)_2$ | |
| 579 | 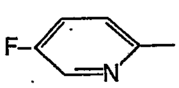 | H | $-CH-CH_2-$<br>$\vert$<br>$CH_3$ | $-N(CH_3)_2$ | |
| 580 | 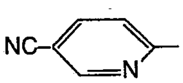 | H | $-CH-CH_2-$<br>$\vert$<br>$CH_3$ | $-N(CH_3)_2$ | |

| Nr. | Z | $R_n$ | Q | A | phys. Daten |
|---|---|---|---|---|---|
| 581 | 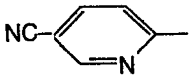 | H | $-(CH_2)_2-$ | $-\underset{\vert \atop CH_3}{N}-C_2H_5$ | |
| 582 | 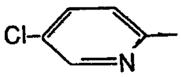 | H | $-CH(CH_3)-$ | $-N(C_2H_5)_2$ | |
| 583 | 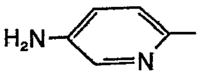 | H | $-(CH_2)_2-$ | $-N(C_2H_5)_2$ | |
| 584 | 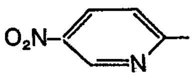 | H | $-(CH_2)_2-$ | $-N(C_2H_5)_2$ | |
| 585 | 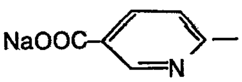 | H | $-(CH_2)_2-$ | $-N(C_2H_5)_2$ | |
| 586 | 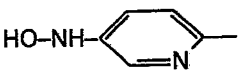 | H | $-(CH_2)_2-$ | $-N(C_2H_5)_2$ | |
| 587 | 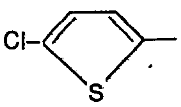 | H | $-(CH_2)_2-$ | $-N(C_2H_5)_2$ | |
| 588 | 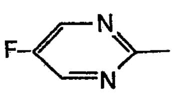 | H | $-(CH_2)_2-$ | $-N(C_2H_5)_2$ | |
| 589 | 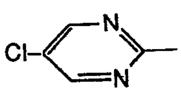 | H | $-(CH_2)_2-$ | $-N(C_2H_5)_2$ | |

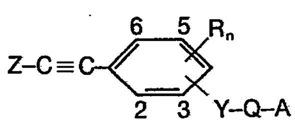

| Nr. | Z | $R_n$ | –Y–Q–A |
|---|---|---|---|
| 701 | $C_6H_5-$ | H | $4-NH-(CH_2)_2-N(C_2H_5)_2$ |
| 702 | $C_6H_5-$ | H | $4-N(CH_3)-(CH_2)_2-N(C_2H_5)_2$ |
| 703 | $C_6H_5-$ | H | $3-NH-(CH_2)_2-N(C_2H_5)_2$ |
| 704 | $C_6H_5-$ | H | $3-N(CH_3)-(CH_2)_2-N(C_2H_5)_2$ |
| 705 | $C_6H_5-$ | H | $2-NH-(CH_2)_2-N(C_2H_5)_2$ |
| 706 | $C_6H_5-$ | H | $2-N(CH_3)-(CH_2)_2-N(C_2H_5)_2$ |
| 707 | $C_6H_5-$ | H | $2-S-(CH_2)_2-N(C_2H_5)_2$ |
| 708 | $C_6H_5-$ | H | $3-S-(CH_2)_2-N(C_2H_5)_2$ |
| 709 | $C_6H_5-$ | H | $4-S-(CH_2)_2-N(C_2H_5)_2$ |

Formulierungsbeispiele

Beispiel 6:

Formulierungsbeispiele für flüssige Wirkstoffe der Formel I (% = Gewichtsprozent)

| a) Emulsionskonzentrate | a) | b) | c) |
|---|---|---|---|
| Wirkstoff | 20% | 40% | 50% |
| Ca-Dodecylbenzolsulfonat | 5% | 8% | 5,8% |
| Ricinusöl-polyäthylenglykoläther (36 Mol AeO) | 5% | – | – |
| Tributylphenoyl-polyäthylenglykoläther (30 Mol AeO) | – | 12% | 4,2% |
| Cyclohexanon | – | 15% | 20% |
| Xylolgemisch | 70% | 25% | 20% |

Aus solchen Konzentraten können durch Verdünnen mit Wasser Emulsionen jeder gewünschten Konzentration hergestellt werden.

| b) Lösungen | a) | b) | c) | d) |
|---|---|---|---|---|
| Wirkstoff | 80% | 10% | 5% | 95% |
| Äthylenglykol-monomethyl-äther | 20% | – | – | – |
| Polyäthylenglykol M G 400 | – | 70% | – | – |
| N-Methyl-2-pyrrolidon | – | 20% | – | – |
| Epoxidiertes Kokosnussöl | – | – | 1% | 5% |
| Benzin (Siedegrenzen 160–190 °C) | – | – | 94% | – |

Die Lösungen sind zur Anwendung in Form kleinster Tropfen geeignet.

| c) Granulate | a) | b) |
|---|---|---|
| Wirkstoff | 5% | 10% |
| Kaolin | 94% | – |
| Hochdisperse Kieselsäure | 1% | – |
| Attapulgit | – | 90% |

Der Wirkstoff wird in Methylenchlorid gelöst, auf den Träger aufgesprüht und das Lösungsmittel anschliessend im Vakuum abgedampft.

| d) Stäubemittel | a) | b) |
|---|---|---|
| Wirkstoff | 2% | 5% |
| Hochdisperse Kieselsäure | 1% | 5% |
| Talkum | 97% | – |
| Kaolin | – | 90% |

Durch inniges Vermischen der Trägerstoffe mit dem Wirkstoff erhält man gebrauchsfertige Stäubemittel.

Beispiel 7:

Formulierungsbeispiele für feste Wirkstoffe der Formel I (% = Gewichtsprozent)

| a) Spritzpulver | a) | b) |
|---|---|---|
| Wirkstoff | 20% | 60% |
| Na-Ligninsulfonat | 5% | 5% |
| Na-Laurylsulfat | 3% | – |
| Na-Diisobutylnaphthalinsulfonat | – | 6% |

| a) Spritzpulver | a) | b) |
|---|---|---|
| Octylphenolpolyäthylenglykoläther 7–8 Mol AeO) | – | 2% |
| Hochdisperse Kieselsäure | 5% | 27% |
| Kaolin | 67% | |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | |
|---|---|
| Wirkstoff | 10% |
| Octylphenolpolyäthylenglykoläther (4–5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 5% | 8% |
| Talkum | 95% | – |
| Kaolin | – | 92% |

Man erhält anwendungsfertige Stäubemittel, indem der Wirkstoff mit dem Träger vermischt und auf einer geeigneten Mühle vermahlen wird.

| d) Extruder Granulat | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff | 3% |
| Polyäthylenglykol | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | |
|---|---|
| Wirkstoff | 40% |
| Äthylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel 8:
Post-emergente Herbizid-Wirkung (Kontaktherbizid)

Sowohl monokotyle wie dikotyle Unkräuter wurden nach dem Auflaufen (in 4- bis 6-Blattstadium) mit einer wässrigen Wirkstoffdispersion in einer Dosierung von 4 kg Wirksubstanz pro Hektar auf die Pflanzen gespritzt und diese bei 24°–26 °C und 45–60% relativer Luftfeuchtigkeit gehalten. Mindestens 15 Tage nach der Behandlung wird der Versuch ausgewertet und das Ergebnis nach folgender Notenskala bewertet:

1: Pflanzen total abgestorben,
2–3: sehr starke Wirkung,
4–6: mittlere Wirkung,
7–8: geringe Wirkung,
9: keine Wirkung (wie unbehandelte Kontrolle).

Post-emergente Wirkung

Aufwandmenge: 4 kg Wirksubstanz/Hektar

| Verb. Nr. | Setaria | Solanum | Sinapis | Stellaria |
|---|---|---|---|---|
| 1 | 2 | 1 | 1 | 1 |
| 402 | 1 | 1 | 1 | 1 |
| 408 | 4 | 1 | 2 | 4 |
| 416 | 2 | 1 | 1 | 1 |
| 417 | 3 | 1 | 1 | 1 |
| 422 | 5 | 1 | 1 | 2 |
| 423 | 6 | 1 | 1 | 3 |
| 431 | 4 | 1 | 1 | 2 |
| 432 | 7 | 2 | 1 | 3 |
| 433 | 3 | 1 | 1 | 1 |
| 438 | 1 | 2 | 1 | 2 |
| 452 | 6 | 1 | 1 | 3 |
| 456 | 7 | 1 | 2 | 3 |
| 471 | 3 | 1 | 1 | 2 |
| 482 | 6 | 1 | 2 | 2 |
| 492 | 4 | 1 | 2 | 3 |
| 493 | 4 | 2 | 2 | 3 |

**Patentansprüche**

1. Aryl-Phenyl-Acetylenverbindungen und deren Ammoniumsalze der Formel I

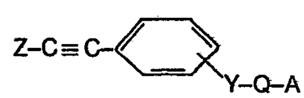

(I)

worin

A eine gegebenenfalls substituierte Aminogruppe,

Q eine $C_2$–$C_6$-Alkylenbrücke,

Y ein Sauerstoff- oder Schwefelatom, oder ein gegebenenfalls substituiertes Stickstoffatom und

Z einen gegebenenfalls substituierten Phenyl- Naphthyl- oder heterocyclischen Rest bedeuten, wobei der Phenylrest mit dem Substituenten –Y–Q–A noch ein bis vier weitere Substituenten tragen kann, mit der Massgabe, dass Z nur dann für einen unsubstituierten Phenylkern steht, wenn der Phenylkern mit dem Substituenten –Y–Q–A einen weiteren Substituenten trägt oder der Substituent –Y–Q–A in der meta-Stellung zur Äthinylbrücke steht oder –Y–Q–A nicht $-O-CH_2-CH_2-N(C_2H^5)_2$ bedeutet.

2. Aryl-Phenyl-Acetylenverbindung gemäss Anspruch 1, der Formel Ia

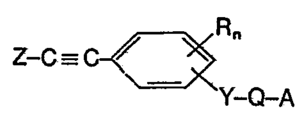

(Ia)

worin

A eine Aminogruppe der Formel

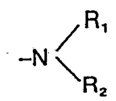 oder 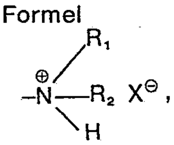

$R_1$ und $R_2$ unabhängig voneinander jeweils Wasserstoff, $C_3$–$C_6$-Alkenyl, $C_3$–$C_6$-Alkinyl, $C_3$–$C_8$-Cycloalkyl oder gegebenenfalls durch Halogen, Hydroxy, $C_1$–$C_4$-Alkoxy, Alkoxycarbonyl mit höchstens 5 Kohlenstoffatomen, Cyan oder Carboxyl substituiertes $C_1$–$C_6$-Alkyl, oder

$R_1$ und $R_2$ zusammen mit dem sie tragenden Stickstoffatom auch einen 5- bis 6-gliedrigen gesättigten Heterocyclus mit insgesamt höchstens 2 Heteroatomen darstellen, der durch $C_1$–$C_3$-Alkyl substituiert sein kann,

$X^{\ominus}$ ein Anion,

Y Sauerstoff, Schwefel oder ein Radikal $-NR_4-$,

$R_4$ Wasserstoff, $C_3$–$C_6$-Alkenyl, $C_3$–$C_6$-Alkinyl, $C_3$–$C_8$-Cycloalkyl oder gegebenenfalls durch Hydroxy, $C_1$–$C_4$-Alkoxy, Alkoxycarbonyl mit höchstens 5 Kohlenstoffatomen, Cyan oder Carboxy substituiertes $C_1$–$C_6$-Alkyl,

Q eine unverzweigte oder verzweigte $C_2$–$C_6$-Alkylenbrücke,

R Wasserstoff, Nitro, Cyan, Trifluormethyl, $C_1$–$C_4$-Alkoxy, $C_1$–$C_4$-Alkylthio, $-NR_5R_6$, $-CO-NR_7R_8$, $-COOR_9$, $-CO-SR_{10}$, Halogen, $-N_3$ oder gegebenenfalls durch $C_1$–$C_4$-Alkyl, Hydroxy, Cyan oder $-COOR_9$ substituiertes $C_1$–$C_4$-Alkyl,

n eine ganze Zahl von 1 bis 4,

$R_5$, $R_7$, $R_8$, $R_9$ und $R_{10}$ unabhängig voneinander jeweils Wasserstoff oder $C_1$–$C_6$-Alkyl, $C_3$–$C_8$-Alkenyl oder $C_3$–$C_8$-Alkinyl,

$R_6$ Wasserstoff, $C_1$–$C_6$-Alkyl, $C_3$–$C_8$-Alkenyl, $C_3$–$C_8$-Alkinyl, $-CO-R_{11}$, $-COO-R_{12}$ oder $-CO-NHR_{13}$ darstellen, wobei

$R_{11}$, $R_{12}$ und $R_{13}$ die gleiche Bedeutung wie $R_4$ haben und

Z für einen gegebenenfalls durch einen oder mehrere Reste, die dieselbe Bedeutung haben wie für R angegeben, oder die Formyl,

$-SO_2-NR_7R_8$, $-NH-NH_2$, $-NHOH$, $-SO-R_9$, $SO_2-R_9$, 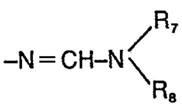 , $COO^{\ominus} M^{\oplus}$

oder gegebenenfalls durch Nitro, Cyano oder $-COOR_9$ substituiertes $C_2$–$C_6$-Alkenyl bedeuten, substituierten Phenyl-, Naphthyl- oder heterocyclischen, aromatischen Rest steht, der mindestens ein Sauerstoff-, Schwefel- oder Stickstoffatom enthält, wobei $M^{\oplus}$ für ein Natrium-, Kalium-, Calcium- oder Magnesium-Kation steht, mit der Massgabe, dass Z dann ein substituierter Phenylrest, ein Naphthyl- oder heterocyclischer Rest sein muss, wenn –Y–Q–A in 4-Stellung zu $Z-C\equiv C-$ steht, A Diäthylamino, R Wasserstoff, Q Äthylen und Y Sauerstoff ist.

3. Aryl-Phenyl-Acetylenverbindungen gemäss Anspruch 2, dadurch gekennzeichnet, dass der Rest –Y–Q–A in der 4-Position zum Äthinylrest steht.

4. Aryl-Phenyl-Acetylenverbindungen gemäss Anspruch 2, dadurch gekennzeichnet, dass der Rest –Y–Q–A in der 3-Position zum Äthinylrest steht.

5. Verbindungen gemäss Anspruch 2, dadurch gekennzeichnet, dass die direkte Alkylenbrücke zwischen dem Brückenatom Y und dem Stickstoffatom der Gruppe A aus 2 oder 3 Kohlenstoffatomen besteht.

6. Verbindungen gemäss Anspruch 2, dadurch gekennzeichnet, dass der Arylrest Z gegebenenfalls substituiertes Phenyl, Furanyl oder Thienyl bedeutet.

7. Verbindungen gemäss Anspruch 2, dadurch gekennzeichnet, dass der Arylrest Z gegebenenfalls substituiertes Pyridyl, Pyrimidinyl, Pyrrolyl, Thiazolyl oder Triazinyl bedeutet.

8. Verbindungen gemäss Anspruch 2, dadurch gekennzeichnet, dass die Reste $R_1$ und $R_2$ für Methyl oder Äthyl stehen.

9. Verbindungen gemäss Anspruch 2, dadurch gekennzeichnet, dass das Brückenglied Y Sauerstoff ist.

10. Verbindungen gemäss Anspruch 2, dadurch gekennzeichnet, dass der Rest –Y–Q–A in der 4-Position zum Aryläthinylrest steht, die direkte Kette zwischen dem Brückenatom Y und dem Stickstoffatom der Gruppe A aus 2 bis 3 Kohlenstoffatomen besteht, der Arylrest Z gegebenenfalls substituiertes Phenyl, Furanyl oder Thienyl bedeutet, das Brückenglied Y Sauerstoff ist und die Reste $R_1$ und $R_2$ für Methyl oder Äthyl stehen.

11. Verbindungen gemäss Anspruch 2, dadurch gekennzeichnet, dass der Rest –Y–Q–A in der 3-Position zum Aryläthinylrest steht, die direkte Kette zwischen dem Brückenatom Y und dem Stickstoffatom der Gruppe A aus 2 bis 3 Kohlenstoffatomen besteht, der Arylrest Z gegebenenfalls substituiertes Phenyl, Furanyl oder Thienyl bedeutet, das Brückenglied Y Sauerstoff ist und die Reste $R_1$ und $R_2$ für Methyl oder Äthyl stehen.

12. Verbindungen gemäss Anspruch 2, dadurch gekennzeichnet, dass der Rest –Y–Q–A in der 4-Position zum Aryläthinylrest steht, die direkte Kette zwischen dem Brückenatom Y und dem Stickstoffatom der Gruppe A aus 2 bis 3 Kohlenstoffatomen besteht, die Arylreste Z gegebenenfalls substituiertes Pyridyl, Pyrimidinyl, Pyrrolyl, Thiazolyl oder Triazinyl bedeuten, das Brückenglied Y Sauerstoff ist und die Reste $R_1$ und $R_2$ für Methyl oder Äthyl stehen.

13. Verbindungen gemäss Anspruch 2, dadurch gekennzeichnet, dass der Rest –Y–Q–A in der 3-Position zum Aryläthinylrest steht, die direkte Kette zwischen dem Brückenatom Y und dem Stickstoffatom der Gruppe A aus 2 bis 3 Kohlenstoffatomen besteht, der Arylrest Z gegebenenfalls substituiertes Pyridyl, Pyrimidinyl, Pyrrolyl, Thiazolyl oder Triazinyl bedeutet, das Brückenglied Y Sauerstoff ist und die Reste $R_1$ und $R_2$ für Methyl oder Äthyl stehen.

14. Verbindungen gemäss einem der Ansprüche 10 bis 13, dadurch gekennzeichnet, dass am zentralen Phenylkern ausser dem Aryläthinylsubstituenten und der Gruppe –Y–Q–A höchstens ein weiterer Substituent aus der Gruppe Halogen, $C_1$–$C_4$-Alkyl, Nitro oder Cyan steht.

15. Die Verbindung 1-[4-(4-Methoxyphenyläthinyl)-phenoxy]-2-diäthylaminoäthan gemäss Anspruch 1.

16. Die Verbindung 1-(3-Phenyläthinyl-phenoxy)-2-diäthylaminoäthan gemäss Anspruch 1.

17. Die Verbindung 1-[4-(4-Fluorphenyläthinyl)-phenoxy]-2-diäthylaminoäthan gemäss Anspruch 1.

18. Die Verbindung 1-(4-Phenyläthinyl-phenoxy)-2-äthylmethylaminoäthan gemäss Anspruch 1.

19. Die Verbindung 1-[4-(2-Thienyläthinyl)-phenoxy]-2-diäthylaminoäthan gemäss Anspruch 1.

20. Die Verbindung 1-Methyl-1-(3-phenyläthinyl-phenoxy)-2-dimethylaminoäthan gemäss Anspruch 1.

21. Die Verbindung 2-Methyl-1-(3-phenyläthinyl-phenoxy)-2-dimethylaminoäthan gemäss Anspruch 1.

22. Verbindungen der Formel IX

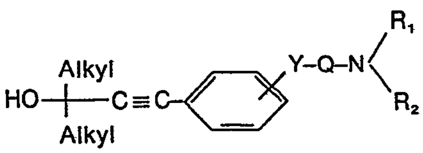 (IX)

worin Y und Q die unter Formel I und $R_1$ und $R_2$ die unter Formel Ia gegebene Bedeutung haben und Alkyl eine $C_1$–$C_4$-Alkylgruppe bedeutet.

23. Verfahren zur Herstellung von Verbindungen der Formel I, und der Ammoniumsalze, gemäss Anspruch 1, dadurch gekennzeichnet, dass man entweder eine Äthinylverbindung der Formel II

$$Z{-}C{\equiv}CH \qquad (II)$$

in Gegenwart eines säurebindenden Mittels und eines Metallkatalysators vorzugsweise unter einer Inertgasatmosphäre mit einem Phenylhalogenid der Formel III

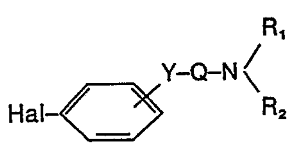 (III)

umsetzt, oder dass man ein aromatisches Halogenid der Formel V

$$Z{-}Hal \qquad (V)$$

in Gegenwart eines säurebindenden Mittels und eines Metallkatalysators vorzugsweise unter einer Inertgasatmosphäre mit einem Phenylacetylen der Formel VI

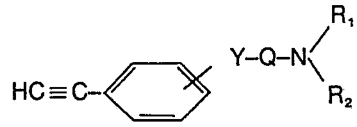 (VI)

worin Q, Y und Z die unter Formel I und $R_1$ und $R_2$ die unter Formel Ia gegebene Bedeutung haben und Hal Brom oder Jod ist, umsetzt und gegebenenfalls in die Ammoniumsalze überführt.

24. Verfahren zur Herstellung der Verbindungen der Formel I und der Ammoniumsalze, gemäss Anspruch 1, dadurch gekennzeichnet, dass man ein aromatisches Halogenid der Formel V

$$Z{-}Hal \qquad (V)$$

in Gegenwart eines säurebindenden Mittels und eines Metallkatalysators gegebenenfalls unter einer Inertgasatmosphäre mit einem Propargylalkohol der Formel VII

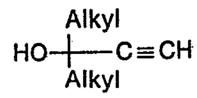 (VII)

umsetzt und die erhaltene Äthinylverbindung der Formel VIII

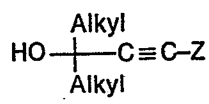 (VIII)

in Gegenwart einer starken Base und eines Metallkatalysators gegebenenfalls unter einer Inertgasatmosphäre mit einem Phenylhalogenid der Formel III

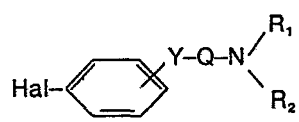 (III)

umsetzt, oder dass man ein Phenylhalogenid der Formel III in Gegenwart eines Metallkatalysators gegebenenfalls unter einer Inertgasatmosphäre zuerst mit dem Propargylalkohol der Formel VII umsetzt und dann die entstandene Äthinylverbindung der Formel IX

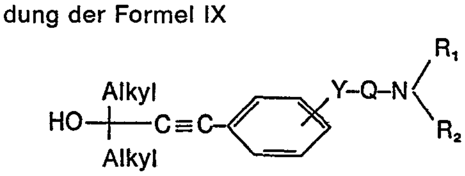 (IX)

in Gegenwart einer starken Base une eines Metallkatalysators gegebenenfalls unter einer Inertgasatmosphäre mit dem aromatischen Halogenid der Formel V, worin Q, Y und Z die unter Formel I und $R_1$ und $R_2$ die unter Formel Ia gegebene Bedeutung haben, Hal Brom oder Jod ist und Alkyl ein $C_1$–$C_4$-Alkylrest ist, umsetzt und gegebenenfalls in die Ammoniumsalze überführt.

25. Verfahren zur Herstellung von Ammoniumsalzen der Verbindung der Formel I, dadurch gekennzeichnet, dass man die freien Aminoverbindungen der Formel I mit einer Säure der Formel HX umsetzt.

26. Herbizides Mittel, dadurch gekennzeichnet, dass es neben Träger- und/oder anderen Zuschlagstoffen als Wirkstoff mindestens eine Aryl-Phenyl-Acetylenverbindung der Formel I, Anspruch 1, enthält.

27. Herstellung eines herbiziden Mittels gemäss Anspruch 26, dadurch gekennzeichnet, dass man Träger- und/oder andere Zuschlagstoffe mit den Wirkstoffen der Formel I innig vermischt und vermahlt.

28. Die Verwendung der Aryl-Phenyl-Acetylenverbindungen der Formel I, Anspruch 1, oder sie enthaltender Mittel zur Bekämpfung von Unkräutern.

29. Die Verwendung gemäss Anspruch 28 zur selektiven postemergenten Bekämpfung von Unkräutern in Nutzpflanzenkulturen.

30. Die Verwendung gemäss Anspruch 29 in Getreide, Mais und Reis.

## Claims

1. An arylphenylacetylene compound or an ammonium salt thereof of the formula

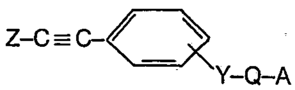

(I)

wherein

A is an unsubstituted or substituted amino group,

Q is a $C_2$–$C_6$alkylene bridge,

Y is an oxygen or a sulfur atom or an unsubstituted or substituted nitrogen atom, and

Z is an unsubstituted or substituted phenyl, naphthyl or heterocyclic radical, which phenyl radical carrying the substituent –Y–Q–A can additionally carry 1 to 4 further substituents, with the proviso that Z can only be an unsubstituted phenyl radical if the phenyl nucleus carrying the substituent –Y–Q–A carries a further substituent or the substituent –Y–Q–A is in the meta-position to the ethynyl bridge or –Y–Q–A is not $-O-CH_2-CH_2-N(C_2H_5)_2$.

2. An arylphenylacetylene compound according to claim 1 of the formula Ia

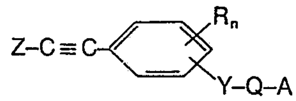

(Ia)

wherein

A is an amino group of the formula

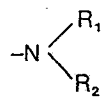

or

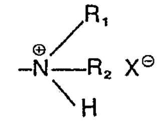

,

$R_1$ and $R_2$, each independently of the other, are hydrogen, $C_3$–$C_6$alkenyl, $C_3$–$C_6$alkynyl, $C_3$–$C_8$cycloalkyl; or $C_1$–$C_6$alkyl, unsubstituted or substituted by halogen, hydroxy, $C_1$–$C_4$alkoxy, alkoxycarbonyl containing at most 5 carbonatoms, cyano or carboxyl; or, together with the nitrogen atom to which they are attached, are also a 5- to 6-membered heterocyclic ring system containing altogether at most 2 hetero atoms and which can be substituted by $C_1$–$C_3$alkyl,

$X^{\ominus}$ is an anion,

Y is oxygen, sulfur or a radical $-NR_4-$, in which $R_4$ is hydrogen, $C_3$–$C_6$alkenyl, $C_3$–$C_6$alkynyl, $C_3$–$C_8$cycloalkyl, or $C_1$–$C_6$alkyl which is unsubstituted or substituted by hydroxy, $C_1$–$C_4$alkoxy, alkoxycarbonyl containing at most 5 carbon atoms, cyano or carboxyl,

Q is a $C_2$–$C_6$alkylene bridge, unbranched or branched ,

R is hydrogen, nitro, cyano, trifluoromethyl, $C_1$–$C_4$alkoxy, $C_1$–$C_4$alkylthio, $-NR_5R_6$, $-CO-NR_7R_8$, $-COOR_9$, $-CO-SR_{10}$, halogen, $-N_3$, or $C_1$–$C_4$alkyl which is unsubstituted or substituted by $C_1$–$C_4$alkyl, hydroxy, cyano or $-COOR_9$,

n is an integer from 1 to 4,

$R_5$, $R_7$, $R_8$, $R_9$ and $R_{10}$, each independently of the other, are hydrogen or $C_1$–$C_6$alkyl, $C_3$–$C_8$alkenyl or $C_3$–$C_8$alkynyl,

$R_6$ is hydrogen, $C_1$–$C_6$alkyl, $C_3$–$C_8$alkenyl, $C_3$–$C_8$alkynyl, $-CO-R_{11}$, $-COOR_{12}$ or $-CO-NHR_{13}$, in which $R_{11}$, $R_{12}$ and $R_{13}$ have the same meanings as $R_4$, and

Z is a phenyl, naphthyl or heterocyclic aromatic radical which contains at least one oxygen, sulfur or nitrogen atom, and which is unsubstituted or substituted by one or more radicals having the same meaning as R or which are formyl,

$-SO_2-NR_7R_8$, $-NH-NH_2$, $-NHOH$, $-SO-R_9$, $SO_2-R_9$, $-N=CH-$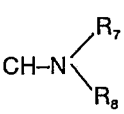, $COO^{\ominus}M^{\oplus}$

or $C_2$–$C_6$alkenyl which is unsubstituted or substituted by nitro, cyano or $-COOR_9$, and $M^{\oplus}$ is a sodium, potassium, calcium or magnesium cation, with the proviso that Z mus be a substituted phenyl radical, or a naphthyl or heterocyclic radical, if –Y–Q–A is in the 4-position to Z–C≡C–, A is diethylamino, R is hydrogen, Q is ethylene and Y is oxygen.

3. An arylphenylacetylene compound according to claim 2, wherein the radical –Y–Q–A is in the 4-position to the ethynyl radical.

4. An arylphenylacetylene compound according to claim 2, wherein the radical –Y–Q–A is in the 3-position to the ethynyl radical.

5. A compound according to claim 2, wherein the direct alkylene bridge between the bridge atom Y and the nitrogen atom of the group A consists of 2 or 3 carbon atoms.

6. A compound according to claim 2, wherein the aryl radical Z is phenyl, furanyl or thienyl, each unsubstituted or substituted.

7. A compound according to claim 2, wherein the aryl radical Z is pyridyl, pyrimidinyl, pyrrolyl, thiazolyl or triazinyl, each unsubstituted or substituted.

8. A compound according to claim 2, wherein $R_1$ and $R_2$ are methyl or ethyl.

9. A compound according to claim 2, wherein the bridge member Y is oxygen.

10. A compound according to claim 2, wherein the radical –Y–Q–A is in the 4-position to the arylethynyl radical, the direct chain between the bridge atom Y and the nitrogen atom of the group A consists of 2 to 3 carbon atoms, the aryl radical Z is phenyl, furanyl or thienyl, each unsubstituted or substituted, the bridge member Y is oxygen, and $R_1$ and $R_2$ are methyl or ethyl.

11. A compound according to claim 2, wherein the radical –Y–Q–A is in the 3-position to the arylethynyl radical, the direct chain between the bridge atom Y and the nitrogen atom of the group A consists of 2 or 3 carbon atoms, the aryl radical Z is phenyl, furanyl or thienyl, each unsubstituted or substituted, the bridge member Y is oxygen, and $R_1$ and $R_2$ are methyl or ethyl.

12. A compound according to claim 2, wherein the radical –Y–Q–A is in the 4-position to the arylethynyl radical, the direct chain between the bridge atom Y and the nitrogen atom of the group A consists of 2 or 3 carbon atoms, the aryl radicals Z is pyridyl, pyrimidinyl, pyrrolyl, thiazolyl or triazinyl, each unsubstituted or substituted, the bridge member Y is oxygen, and $R_1$ and $R_2$ are methyl or ethyl.

13. A compound according to claim 2, wherein the radical –Y–Q–A is in the 3-position to the arylethynyl radical, the direct chain between the bridge atom Y and the nitrogen atom of the group A consists of 2 or 3 carbon atoms, the aryl radical Z is pyridyl, pyrimidinyl, pyrrolyl, thiazolyl or triazinyl, each unsubstituted or substituted, the bridge member Y is oxygen, and $R_1$ and $R_2$ are methyl or ethyl.

14. A compound according to any one of claims 10 to 13 which carries at the central phenyl nucleus, in addition to the arylethynyl substituent and the group –Y–Q–A, at most one further substituent selected from the group consisting of halogen, $C_1$–$C_4$alkyl, nitro or cyano.

15. 1-[4-(4-Methoxyphenylethynyl)phenoxy]-2-diethylaminoethane according to claim 1.

16. 1-(3-Phenylethynylphenoxy)-2-diethylaminoethane according to claim 1.

17. 1-[4-(4-Fluorophenylethynyl)phenoxy]-2-diethylaminoethane according to claim 1.

18. 1-(4-Phenylethynylphenoxy)-2-ethylmethylaminoethane according to claim 1.

19. 1-[4-(2-Thienylethynyl)phenoxy]-2-diethylaminoethane according to claim 1.

20. 1-Methyl-1-(3-phenylethynylphenoxy)-2-dimethylaminoethane according to claim 1.

21. 2-Methyl-1-(3-phenylethynylphenoxy)-2-dimethylaminoethane according to claim 1.

22. A compound of the formula IX

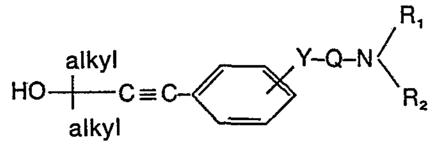

(IX)

wherein Y and Q are as defined for formula I and $R_1$ and $R_2$ are as defined for formual Ia, and alkyl denotes a $C_1$–$C_4$alkyl group.

23. A process for the production of a compound of the formula I, or an ammonium salt thereof, according to claim 1, which process comprises reacting either an ethynyl compound of the formula II

$$Z\text{–}C\equiv CH \qquad (II)$$

in the presence of an acid acceptor and of a metal catalyst, preferably in an inert gas atmosphere, with a phenyl halide of the formula III

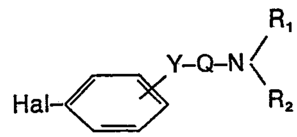

(III)

or reacting an aromatic halide of the formula V

$$Z\text{–}Hal \qquad (V)$$

in the presence of an acid acceptor and of a metal catalyst, in an inert gas atmosphere, with a phenylacetylene of the formula VI

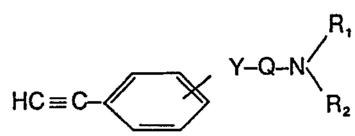

(VI)

wherein Q, Y and Z are as defined for formula I and $R_1$ and $R_2$ are as defined for formula Ia, and Hal is bromine or iodine, and, if desired, converting the resultant free compound into the ammonium salt.

24. A process for the production of a compound of the formula I, or an ammonium salt thereof, according to claim 1, which process comprises reacting an aromatic halide of the formula V

$$Z\text{–}Hal \qquad (V)$$

in the presence of an acid acceptor and of a metal catalyst, optionally in an inert gas atmosphere, with a propargyl alcohol of the formula VII

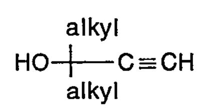

(VII)

and reacting the resultant ethynyl compound of the formula VIII

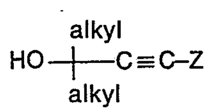

(VIII)

in the presence of a strong base and of a metal catalyst, optionally in an inert gas atmosphere, with a phenyl halide of the formula III

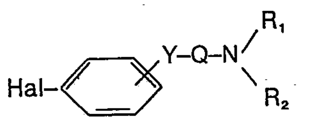

(III)

or reacting a phenyl halide of the formula III, in the presence of a metal catalyst and optionally in an inert gas atmosphere, initially with the propargyl alcohol of the formula VII and then reacting the resultant ethynyl compound of the formula IX

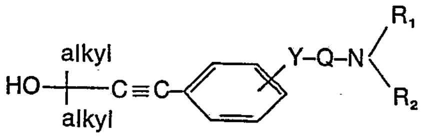

(IX)

in the presence of a strong base and of a metal catalyst, optionally in an inert gas atmosphere, with the aromatic halide of the formula V, in which formulae Q, Y and Z are as defined for formula I and $R_1$ and $R_2$ are as defined for formula Ia, Hal is bromine or iodine, and alkyl denotes a $C_1$–$C_4$alkyl radical, and, if desired, converting the resultant free compound into the ammonium salt.

25. A process for the production of an ammonium salt of the compound of the formula I, which process comprises reacting the free amino compound of the formula I with an acid of the formula HX.

26. A herbicidal composition which contains, as active ingredient, at least one arylphenylacetylene compound of the formula I according to claim 1, together with carriers and/or other adjuvants.

27. A process for the production of a herbicidal composition according to claim 26, which process comprises homogeneously mixing and grinding carriers and/or other adjuvants with a compound of the formula I.

28. A method of controlling weeds which comprises the use of an arylphenylacetylene compound of the formula I according to claim 1 or of a composition containing such a compound.

29. The method of claim 28 for the selective postemergence control of weeds in crops of useful plants.

30. The method of claim 29 for the selective postemergence control of weeds in crops of cereals, maize and rice.

**Revendications**

1. Composés aryl-phényl-acétylène et leurs sels d'ammonium de formule I

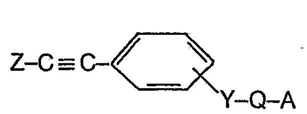

, (I)

où

A représente un groupe amino éventuellement substitué

Q représente un pont alcoylène en $C_2$ à $C_6$,

Y représente un atome d'oxygène ou de soufre, ou un atome d'azote éventuellement substitué et

Z représente un radical phényl-naphthyl- ou hétérocyclique éventuellement substitué, où le radical phényle avec le substituant –Y–Q–A peut encore porter de 1 à 4 autres substituants, avec la précision que Z ne peut représenter un noyau phényle non substitué que lorsque le noyau phényle avec le substituant –Y–Q–A porte un autre substituant ou le substituant –Y–Q–A est en position méta par rapport au pont éthynyle ou –Y–Q–A ne représente pas $-O-CH_2-CH_2-N(C_2H_5)_2$.

2. Composés aryl-phényl-acétylène selon la revendication 1, de formule Ia

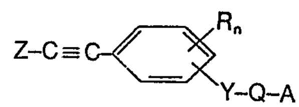

(Ia)

où

A représente un groupe amino de formule

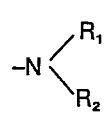

ou

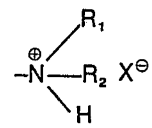

,

$R_1$ et $R_2$ représentent chacun indépendamment l'un de l'autre un hydrogène, un alcényle en $C_3$ à $C_6$, un alcynyle en $C_3$ à $C_6$, un cycloalcoyle en $C_3$ à $C_8$ où un alcoyle en $C_1$ à $C_6$ éventuellement substitué par un halogène, un hydroxy, un alcoxy en $C_1$ à $C_4$, un alcoxycarbonyle avec au plus 5 atomes de carbone, un xyano ou un carboxyle, ou

$R_1$ et $R_2$ représentent également ensemble avec l'atome d'azote qui les porte un hétérocycle saturé à 5 à 6 chaînons avec au total au plus 2 hétéroatomes, qui peut être substitué par un alcoyle en $C_1$ à $C_3$,

$X^{\ominus}$ représente un anion,

Y représente un oxygène, un soufre ou un radical $-NR_4-$,

$R_4$ représente un hydrogène, un alcényle en $C_3$ à $C_6$, un alcynyle en $C_3$ à $C_6$, un cycloalcoyle en $C_3$ à $C_8$ ou un alcoyle en $C_1$ à $C_6$ éventuellement substitué par un hydroxy, un alcoxy en $C_1$ à $C_4$, un alcoxycarbonyle avec au plus 5 atomes de carbone, un cyano ou un carboxyle,

Q représente un pont alcoylène en $C_2$ à $C_6$ non ramifié ou ramifié,

R représente un hydrogène, un nitro, un cyano, un trifluorométhyle, un alcoxy en $C_1$ à $C_4$, un alcoylthio en $C_1$ à $C_4$, $-NR_5R_6$, $-CO-NR_7R_8$, $-COOR_9$, $-CO-SR_{10}$, un halogène, $-N_3$ ou un alcoyle en $C_1$ à $C_4$ éventuellement substitué par un alcoyle en $C_1$ à $C_4$, un hydroxy, un cyano ou $-COOR_9$,

n représente un nombre entier allant de 1 à 4,

$R_5$, $R_7$, $R_8$, $R_9$ et $R_{10}$ représentent chacun indépendamment l'un de l'autre n hydrogène ou un alcoyle en $C_1$ à $C_6$, un alcényle en $C_3$ à $C_8$ ou un alcynyle en $C_3$ à $C_8$,

$R_6$ représente un hydrogène, un alcoyle en $C_1$ à $C_6$, un alcényle en $C_3$ à $C_8$, un alcynyle en $C_3$ à $C_8$, $-CO-R_{11}$, $-COO-R_{12}$ ou $-CO-NHR_{13}$, où

$R_{11}$, $R_{12}$ et $R_{13}$ ont la même signification que $R_4$ et

Z représente un alcényle en $C_2$ à $C_6$ éventuellement substitué par un ou plusieurs radicaux qui ont la même signification que celle indiquée pour R, ou un formyle,

$$-SO_2-NR_7R_8,\ -NH-NH_2-NHOH,\ -SO-R_9,\ SO_2-R_9,\ -N{=}CH-N\begin{matrix}R_7\\R_8\end{matrix},\ COO^{\ominus}M^{\oplus}$$

ou un alcényle en $C_2$ à $C_6$ éventuellement substitué par un nitro, un cyano ou $-COOR_9$, un radical aromatique phényle, naphtyle ou hétérocyclique substitué, qui contient au moins un atome d'oxygène, de soufre ou d'azote, où $M^{\oplus}$ représente un cation sodium, potassium, calcium ou magnésium, avec la précision que Z doit être un radical phényle, naphtyle ou hétérocyclique lorsque –Y–Q–A est en position 4 par rapport à $Z-C{\equiv}C-$, A est un diéthylamino, R un hydrogène, Q un éthylène et Y un oxygène.

3. Composés aryl-phényl-acétylène selon la revendication 2, caractérisés en ce que le radical –Y–Q–A est en position 4 par rapport au radical éthynyle.

4. Composés aryl-phényl-acétylène selon la revendication 2, caractérisés en ce que le radical –Y–Q–A est en position 3 par rapport au radical éthynyle.

5. Composés selon la revendication 2, caractérisés en ce que le pont alcoylène direct entre l'atome de pont Y et l'atome d'azote du groupe A se compose de 2 ou 3 atomes de carbone.

6. Composés selon la revendication 2, caractérisés en ce que le radical aryle Z représente un phényle, furanyle ou thiényle éventuellement substitué.

7. Composés selon la revendication 2, caractérisés en ce que le radical aryle Z représente un pyridyle, pyrimidinyle, pyrrolyle, thiazolyle ou triazinyle éventuellement substitué.

8. Composés selon la revendication 2, caractérisés en ce que les radicaux $R_1$ et $R_2$ représentent un méthyle ou un éthyle.

9. Composés selon la revendication 2, caractérisés en ce que l'élément de pont Y est un oxygène.

10. Composés selon la revendication 2, caractérisés en ce que le radical –Y–Q–A est en position 4 par rapport au radical aryléthynyle, la chaîne directe entre l'atome de pont Y et l'atome d'azote du groupe A se compose de 2 à 3 atomes de carbone, le radical aryle Z représente un phényle, furanyle ou thiényle éventuellement substitué, l'élément de pont Y est un oxygène et les radicaux $R_1$ et $R_2$ représentent un méthyle ou un éthyle.

11. Composés selon la revendication 2, caractérisés en ce que le radical –Y–Q–A est en position 3 par rapport au radical aryléthynyle, la chaîne directe entre l'atome de pont Y et l'atome d'azote du groupe A se compose de 2 à 3 atomes de carbone, le radical aryle Z représente un phényle, furanyle ou thiényle éventuellement substitué, l'élément de pont Y est un oxygène et les radicaux $R_1$ et $R_2$ représentent un méthyle ou un éthyle.

12. Composés selon la revendication 2, caractérisés en ce que le radical –Y–Q–A est en position 4 par rapport au radical aryléthynyle, la chaîne directe entre l'atome de pont Y et l'atome d'azote du groupe A se compose de 2 à 3 atomes de carbone, les radicaux aryle Z représentent un pyridyle, un pyrimidinyle, un pyrrolyle, un thiazolyle ou un triazinyle éventuellement substitués, l'élément de pont Y est un oxygène et les radicaux $R_1$ et $R_2$ représentent un méthyle ou un éthyle.

13. Composés selon la revendication 2, caractérisés en ce que le radical –Y–Q–A est en position 3 par rapport au radical aryléthynyle, la chaîne directe entre l'atome de pont Y et l'atome d'azote du groupe A se compose de 2 à 3 atomes de carbone, le radical aryle Z représente un pyridyle, pyrimidinyle, pyrrolyle, thiazolyle ou triazinyle éventuellement substitué, l'élément de pont Y est un oxygène et les radicaux $R_1$ et $R_2$ représentent un méthyle ou un éthyle.

14. Composés selon l'une des revendications 10 à 13, caractérisés en ce que sur le noyau phényle central, outre le substituant aryléthynyle et le groupe –Y–Q–A se trouve au plus un autre substituant du groupe halogène, alcoyle en $C_1$ à $C_4$, nitro ou cyano.

15. Le composé 1-[4-(4-méthoxyphényléthynyl)-phénoxy]-2-diéthyl-aminoéthane selon la revendication 1.

16. Le composé 1-(3-phényléthynyl-phénoxy)-2-diéthylaminoéthane selon la revendication 1.

17. Le composé 1-[4-(4-fluorophényléthynyl)-phénoxy]-2-diéthyl-aminoéthane selon la revendication 1.

18. Le composé 1-(4-phényléthynyl-phénoxy)-2-éthylméthyl-aminoéthane selon la revendication 1.

19. Le composé 1-[4-(2-thiényléthynyl)-phénoxy]-2-diéthyl-aminoéthane selon la revendication 1.

20. Le composé 1-méthyl-1-(3-phényléthynyl-phénoxy)-2-diméthyl-aminoéthane selon la revendication 1.

21. Le composé 2-méthyl-1-(3-phényléthynyl-phénoxy)-2-diméthylaminoéthane selon la revendication 1.

22. Composés de formule IX

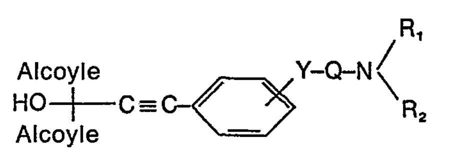

(IX)

où Y et Q ont la signification donnée pour la formule I et $R_1$ et $R_2$ ont la signification donnée pour la formule Ia et alcoyle représente un groupe alcoyle en $C_1$ à $C_4$.

23. Procédé de préparation de composés de formule I, et des sels d'ammonium selon la revendication 1, caractérisé en ce qu'ou bien on fait réagir un composé éthynyle de formule II

Z–C≡CH (II)

en présence d'un agent liant acide et d'un catalyseur métallique de préférence sous une atmosphère de gaz inerte avec un halogénure de phényle de formule III

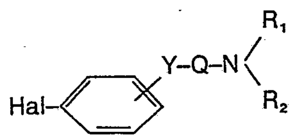
(III)

ou bien on fait réagir un halogénure aromatique de formule V

Z–Hal (V)

en présence d'un agent liant acide et d'un catalyseur métallique de préférence sous une atmosphère de gaz inerte avec un phénylacétylène de formule VI

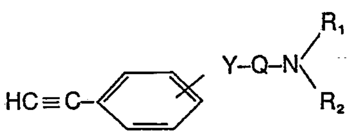
(VI)

où, Q, Y et Z ont la signification donnée pour la formule I et $R_1$ et $R_2$ ont la signification donnée pour la formule Ia et Hal est un brome ou un iode, et éventuellement on transforme en les sels d'ammonium.

24. Procédé de préparation des composés de formule I et des sels d'ammonium selon la revendication 1, caractérisé en ce qu'on fait réagir un halogénure aromatique de formule V

Z–Hal (V)

en présence d'un agent liant acide et d'un catalyseur métallique éventuellement sous une atmosphère de gaz inerte avec un propargylalcool de formule VII

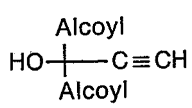
(VII)

et en ce qu'on fait réagir le composé éthynyle obtenu de formule VIII

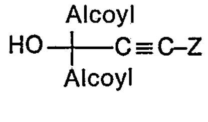
(VIII)

en présence d'une base forte et d'un catalyseur métallique éventuellement sous une atmosphère de gaz inerte avec un phénylhalogénure de formule III

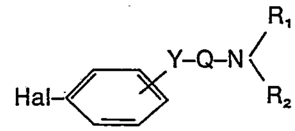
(III)

ou en ce qu'on fait réagir un phénylhalogénure de formule III en présence d'un catalyseur métallique éventuellement sous une atmosphère de gaz inerte tout d'abord avec le propargylalcool de formule VII puis en ce qu'on fait réagir le composé éthynyle apparu de formule IX

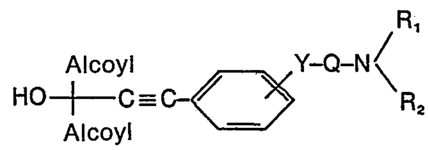
(IX)

en présence d'une base forte et d'un catalyseur métallique éventuellement sous une atmosphère de gaz inerte avec l'halogénure aromatique de formule V, où Q, Y et Z ont la signification donnée pour la formule I er $R_1$ et $R_2$ ont la signification donnée pour la formule Ia, Hal est un brome ou un iode et alcoyle est un radical alcoyle en $C_1$ à $C_4$, et éventuellement en ce qu'on transforme en les sels d'ammonium.

25. Procédé de préparation de sels d'ammonium du composé de formule I, caractérisé en ce qu'on fait réagir les composés amino libres de formule I avec un acide de formule HX.

26. Agent herbicide caractérisé en ce qu'il contient, outre des supports et/ou autres additifs, comme matière active au moins un composé arylphényl-acétylène de formule I de la revendication 1.

27. Préparation d'un agent herbicide selon la revendication 26, caractérisée en ce qu'on mélange intimement des supports et/ou autres additifs avec les matières actives de formule I et en ce qu'on broie.

28. Application des composés aryl-phénylacétylène de formule I de la revendication 1 ou des agents qui les contiennent à la lutte contre les mauvaises herbes.

29. Application selon la revendication 28 à la lutte sélective en post-levée contre les mauvaises herbes dans les cultures de plantes utiles.

30. Application selon la revendication 29 dans les céréales, le maïs et le riz.